# EUROPEAN PATENT SPECIFICATION

(11) **EP 3 818 046 B1**
(45) Date of publication and mention of the grant of the patent: **10.05.2023**
(21) Application number: 19736341.9
(22) Date of filing: 01.07.2019
(51) Int. Cl.: C07D 217/22, C07D 217/02, A61K 31/472, A61P 25/16, A61P 25/28

(54) **LACTATE ENHANCING COMPOUNDS AND USES THEREOF**
LACTATVERSTÄRKENDE VERBINDUNGEN UND VERWENDUNGEN DAVON
COMPOSÉS AMÉLIORANT LE LACTATE ET LEURS UTILISATIONS

(30) Priority: 02.07.2018 EP 18181080
(43) Date of publication of application: 12.05.2021
(73) Proprietor: ECOLE POLYTECHNIQUE FEDERALE DE LAUSANNE (EPFL), 1015 Lausanne (CH); Gliapharm SA, 1202 Genève (CH)
(72) Inventor: MAGISTRETTI, Pierre, Thuwal, 23955-6900 (SA); LENGACHER, Sylvain, 1000 Lausanne 26 (CH); FINSTERWALD, Charles, 1255 Veyrier (CH)
(74) Representative: reuteler & cie SA
(86) International application number: PCT/EP2019/067631
(87) International publication number: WO 2020/007807

(56) References cited:
- EP-A1- 2 586 772
- WO-A1-00/09495
- WO-A1-01/58899
- WO-A1-2004/080464
- WO-A1-2017/068583
- WO-A2-2007/076092
- JP-A- 2012 072 068

## Description

### Field of the Invention

The present invention relates generally to the field of lactate enhancing agents and in particular the use of lactate enhancing agents for the treatment of neurological disorders, comprising neurodegenerative and psychiatric diseases.

### Background of the Invention

Neurodegenerative diseases represent some of the pathologies with the highest unmet needs. They are expected to become the first cause of death by 2050. These pathologies are complex and difficult to treat. Finding treatments has been a challenge for the pharmaceutical industry for the past decades, but limited progress has been made in the field. Most of the therapeutic strategies so far have aimed at targeting neurons directly, using a 'neuro-centric' approach, largely letting aside the important role of other cell types of the nervous system, including astrocytes.

Astrocytes outnumber neurons in the brain and together with oligodendrocytes and microgliocytes form a category of cells called glial cells that support neuronal activity and survival. In the past decade, considerable attention has been focused on understanding the role of astrocytes in physiological processes, as well as their implication in the development of neurological diseases including neurodegenerative disorders, age-related cognitive impairments and psychiatric diseases. While glial cells were though for a long time to only be important for nervous tissue structural support - a sort of brain 'glue' -, their much more important role for the control of fundamental processes has now been largely acknowledged. In particular, astrocytes play a fundamental role by providing energy to neurons, which is required for their function - transmit electrical information - and survival. Hence, astrocytes were found to be key for numerous brain physiological processes that include neuronal protection, neuronal function, synaptic plasticity, and memory consolidation (Magistretti et al., 2018, Nat. Rev. Neurosci., 19(4):235-249*).*

Although neurodegenerative diseases have historically been considered as pathologies that exclusively result from neuronal death, it has become clear that other cell types, such as astrocytes, contribute to these pathologies. A large body of evidence has linked astrocytes activity to mild cognitive impairments (MCI), Alzheimer's disease (AD), amyotrophic lateral sclerosis (ALS), depression and others. For instance, in AD patients, activated astrocytes are preferentially located in the vicinity of amyloid plaques, where they exhibit abnormal morphology and mitochondrial function. In the early stage of the disease, activated astrocytes have neuroprotective action by internalizing and degrading amyloid plaques, while upon progression of the disease, deposit of amyloid plaques leads to astrocytic death that in turn results in further amyloid accumulation (Nagele et al.2004, Neurobiol. Aging, 25(5):663-74). There are clear indications that age-related changes of astrocytes have a role in the development of age-related neurodegenerative disorders, such as MCI and AD (Cai et al., 2017, J. Neurol. 264(10):2068-74). Astrocytosis is a typical morphological feature of the AD brain and represents either proliferation of astrocytes, in an effort to support dying neurons, or a reaction to degrade the increasing amounts of toxic β-amyloid peptides. Of particular interest, exposure of astrocytes to β Amyloid in vitro alters their metabolic activity thus resulting in reduction of the neuronal protection against oxidative stresses (Allaman et al., 2010, J. Neurosci 30(9):3326-38*).*

Deregulation of brain energy metabolism is an important contributor to the development of neurodegenerative diseases and age-associated cognitive decline. These disorders have been linked to decreased mitochondrial activity, increased oxidative stress and diminished cerebral glucose metabolism. For instance, glucose hypometabolism in the brain appears early in the genesis of AD and in fact represents a common phenomenon with other neurodegenerative diseases (Yin et al, 2016, Free Radic. Biol. Med., 100:108-22*;* Fu et al. 2014, Biogerontology, 15(6):579-86*;* Demetrius et al, 2013, Biogerontology, 14(6): 641-9*;* Demetrius et al., 2014, front Physiol 5:522*;* Tomi et al., 2013, Brain Res., 1495:61-75*;* Ferreira et al., 2010, Curr Drug Targets, 11(10):1193-2016). Mitochondrial dysfunction, which is associated with age-related neurodegeneration, is particularly prevalent in AD (Beal, 2005, Neurobiol Aging, 26(5):585-6; Yao et al., 2011, Curr Pharm Des 17(31):3474-9). Studies on patients with AD and mouse models have highlighted the down regulation of several cerebral genes involved in energy regulation (Liang et al., 2008, Proc. Natl. Acad. Sci. USA, Mar 18;105(11):4441-6). As a result, significant correlation between diminished cerebral glucose metabolism and cognitive performance has been shown in AD patients (Thomas et al., 2015, J. Nutr. Health Aging, 19(1):58-63*;* Woo et al, 2010, Int. J. Geriatr. Psychiatry, 25(11):1150-8)*.* Together, these data indicate that astrocytes activity and metabolic coupling that are impaired in MCI and AD may result in the characteristic accumulation of amyloid plaques and neuronal degeneration in specific brain areas.

In ALS, deficit of mitochondrial activity and energy production were found to be responsible, at least in part, for motor neuron degeneration (Boillee et al., 2006, Neuron, 52:39-59). Astrocytes may also play an important role through the regulation of glutamate uptake, which is dramatically impaired in ALS (Rothstein et al., 1990, Ann. Neurol., 28:18-25*;* Spreux-Varoquaux et al., 2002, J. Neurol. Sci., 193:73-78)*.*

Other neurological pathologies including psychiatric disorders such as depression also exhibit impairments in brain energy metabolism (Elsayed and Magistretti, 2015, Front Cell Neurosci, 9:468). In addition, growing evidence indicates that alterations of glial cells also contribute to the pathophysiology and treatment of major depression (Rajkowska and Stockmeier, 2013, Curr Drug Targets 14, 1225-1236*;* Czeh et al., 2006, Neuropsychopharmacology 31, 1616-1626*;* Banasr et al., 2010, Mol Psychiatry 15, 501-511*;* Banasr and Duman, 2008, Biol Psychiatry 64, 863-870)*.* ^{∗} Combinations of lactate and CXRC4 antagonists have been proposed for the treatment of neurodegenerative disease (WO 2017/068583).

Interestingly, a specific metabolite of glucose, i.e. lactate, appears to play a particularly important role in astrocyte-neuron metabolic coupling. Indeed, lactate that is produced by astrocytes is used by neurons as preferential energy source upon neuronal activity, through the so-called astrocyte-neuron lactate shuttle (ANLS) (Pellerin et al., 2012, J, Cereb Blood Flow Metab., 32(7):1152-66). Lactate is produced in astrocytes through the process of aerobic glycolysis, i.e. the transformation of glucose into lactate in the presence of oxygen, a process usually better known to occur in the absence of oxygen to produce energy (e.g. in muscles during physical activity). The source of glucose in the brain can either come from the circulation (astrocyte feet are in close contact with capillaries) or from internal stores of glycogen (cerebral glycogen is exclusively present in astrocytes). Upon synaptic activity, lactate is produced by astrocytes and transferred to neurons, where it is transformed into pyruvate to enter the Tricarboxylic acid (TCA) cycle and produce ATP. In this context, lactate was shown to act as a neuroprotective agent against glutamate-mediated excitotoxicity (Jourdain et al., 2016, Sci. Rep., 6:21250), as well as against cerebral ischemia *in vivo* (Berthet et al., 2012, Cerebrovasc Dis., 34(5-6):329-35). In addition to its neuroprotective effects, ANLS was found to be key in the regulation of long-term memory consolidation *(*Suzuki et al, 2011, Cell 144(5):810-23*),* as well as in the regulation of the expression of genes that modulate synaptic function and plasticity (Yang et al., 2014, Proc Natl Acad Sci USA, 111(33):12228-33*;* Tadi et al. 2015, PLoS One, 10(10):e0141568). Lactate indeed not only plays a key role in providing energy to neurons, but also acts as a regulator of synaptic plasticity through signalling activities (Magistretti and Allaman, 2018, Nat Rev Neurosci 19(4):235-249)*.* Furthermore, the transport of lactate was found to be impaired in a mouse model of ALS, as well as in the nervous system of ALS patients (Lee et al., 2012, Nature, 487(7408):443-8), providing additional evidence for the role of lactate in neurodegenerative disease. In addition, administration of lactate was found to produce antidepressant like effects in a number of animal models of depression (Carrard et al., 2018, Mol Psychiatry, 23(2):488). ^{∗}Isoquinolines derivatives have been described with angiogenesis inhibiting activity (WO 00/09495).

Given the critical role of ANLS in neuronal protection and cognition and the observed impairment of astrocytes function and brain energy metabolism in a number of neurological diseases including MCI, AD, ALS and depression, there is an emerging need to develop lactate enhancing drugs.

### Summary of the Invention

The scope of the invention and thus of protection is defined by the claims and does not extend beyond their scope, even if the following summary and detailed description were to include subject-matter not encompassed by the claims. In particular, the invention does not include any therapeutic/ medical treatment of the human or animal body, even in such subject-matter is disclosed or implied in the following description.

The present invention is based on the unexpected findings of new molecules stimulating release of lactate and glycogenolysis in primary astrocytes cell cultures in vitro and in mice *in vivo* and having neuroprotective effects in a mouse model of ALS and mnemonic effect in a mouse model of memory.

A first aspect of the invention provides a compound of the invention, as well as tautomers, geometrical isomers, optically active forms, enantiomeric mixtures thereof, pharmaceutically acceptable salts, pharmaceutically active derivative and mixtures thereof.

According to another aspect of the invention, is provided a compound of the invention for use as a medicament.

According to another aspect of the invention, is provided a pharmaceutical comprising at least one compound according to the invention and a pharmaceutically acceptable carrier, diluent or excipient thereof as defined herein.

According to another aspect, the invention provides a compound of the invention for use in the prevention and/or treatment of a disorder or a disease associated with an abnormally low intracerebral energy metabolism or in the central nervous system and/or a neurological disorder comprising a neurodegenerative disorder or a psychiatric disorder.

According to another aspect, the invention provides a compound of the invention for use in the prevention and/or treatment of cognitive impairments related to ageing such as, but not limited to, age-associated cognitive decline and age-related memory impairments and for enhancing cognitive and memory functions in healthy subjects.

According to another, the invention provides a use of a compound of the invention as well as tautomers, geometrical isomers, optically active forms, enantiomeric mixtures thereof, pharmaceutically acceptable salts, pharmaceutically active derivative and mixtures thereof for the preparation of a pharmaceutical composition for the prevention and/or treatment of a disorder or a disease associated with an abnormal energy metabolism in the central nervous system and/or a neurodegenerative disorder.

According to another, the invention provides a method of preventing or treating a disorder or a disease associated with an abnormally low intracerebral energy metabolism or in the central nervous system and/or a neurodegenerative disorder in a subject, said method comprising administering in a subject in need thereof a therapeutically effective amount of a compound of the invention, a tautomer, a geometrical isomer, an optically active form, an enantiomeric mixture, a pharmaceutically acceptable salt, a pharmaceutically active derivative thereof or a mixture thereof.

Also disclosed is a method of increasing the intracerebral lactate levels in a subject, said method comprising administering in a subject in need thereof an effective amount of a compound of the invention or a tautomer, a geometrical isomer, an optically active form, an enantiomeric mixture, a pharmaceutically acceptable salt, a pharmaceutically active derivative thereof or a mixture thereof to induce increased intracerebral lactate levels.

A method for enhancing cognitive and memory functions in a subject, said method comprising administering an effective amount of a compound of a compound of the invention or a tautomer, a geometrical isomer, an optically active form, an enantiomeric mixture, a pharmaceutically acceptable salt, a pharmaceutically active derivative thereof or a mixture thereof.

According to another aspect, is provided a process for the preparation of a compound according to Formula (I) comprising the step of reacting an aniline intermediate of Formula **(iii)** with an isoquinoline intermediate of Formula **(ii)** in a polar solvent to a compound of Formula (Ia).

According to another aspect, is provided a process for the preparation of a compound according to Formula (I) comprising the step of reducing a carbonyl intermediate of Formula **(viii)** to lead to a compound of Formula (Iab).

### Description of the figures

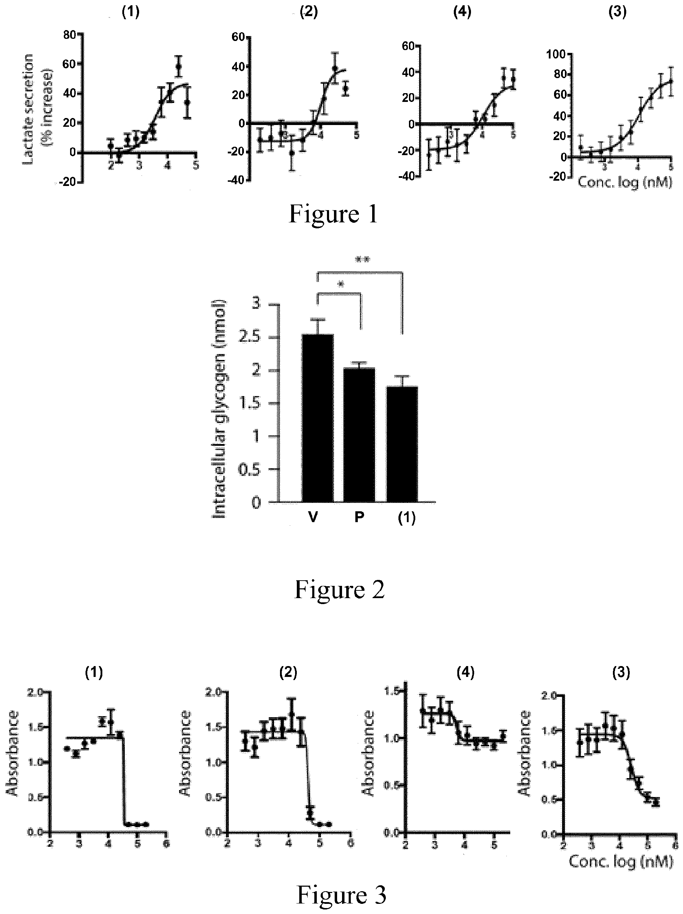

**Figure 1** shows the lactate release from primary cultures of astrocytes measured at 90 min after stimulation with compounds of the invention **(1)** to **(4)** at concentrations ranging from 100 nM to 100 µM as described in Example 2, represented as % of positive control effect (carbonyl cyanide m-chlorophenyl hydrazone (CCCP), 2µM) ± SEM; n= 9.
**Figure 2** represents intracellular levels of glycogen in astrocytes 3 hours after stimulation with Compound **(1)** (10 µM) as described in Example 2, compared to that of Vehicle alone (V) or a positive control known stimulate degradation of glycogen through the activation of glycogen phosphorylase (P; 10 µM), represented as the mean of intracellular glycogen levels (nmoles) ± SEM; n=6; * p<0.05; ** p<0.01.
**Figure 3** shows *in vitro* mitochondrial activity of primary astrocytes measured as described in Example 2 at 24 hours after treatment with compounds **(1)** to **(4)** of the invention at concentrations ranging from 100 nM to 200 µM, represented as mean absorbance of MTT colorimetric assay ± SEM; n=9.

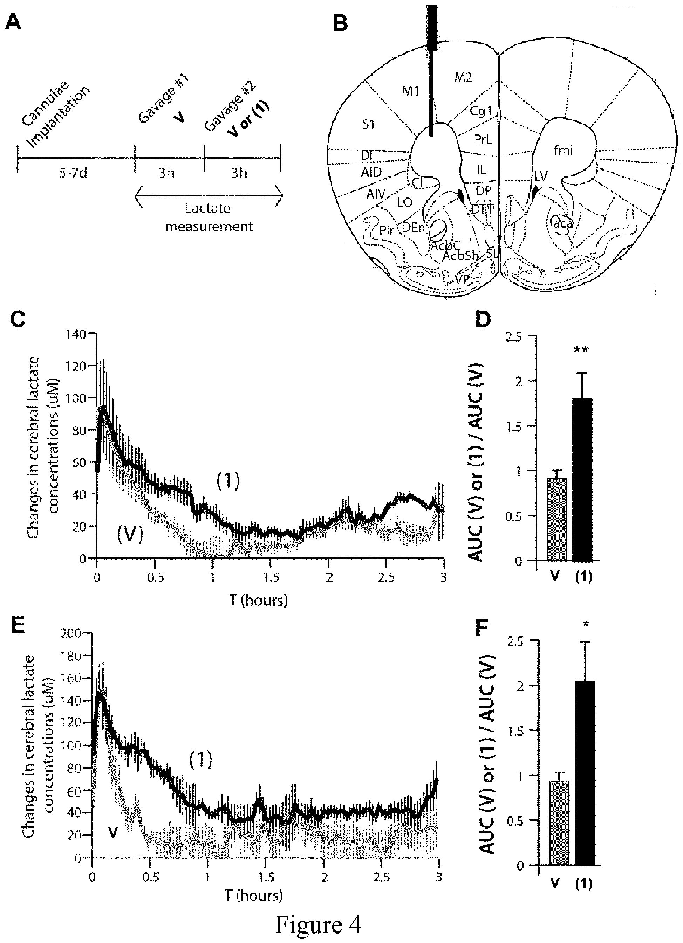

**Figure 4** shows the monitoring of cerebral lactate production as described in Example 2. **A:** experimental schedule with surgical implantation of cerebral cannulae 5 to 7 days prior administration of Vehicle (V) followed, 3h later by administration of (V) or compound invention **(1)** of the invention for another 3 hours (10 mg/kg or 100 mg/kg); **B:** Localization of the lactate probe implanted in mouse brain; **C,E**: Fluctuations of intracerebral lactate levels versus time (T) recording after administration of (V), followed 3h later by (V), **(1)** at 10 mg/kg **(C)** or **(1)** at 100 mg/kg **(E)**; **D,F**: Area under curve (AUC) calculated from lactate fluctuations shown in (C) and (D). Ratio of AUC after 2^{nd} gavage with (V) or **(1)** at 10 mg/kg **(D)**, as well as (V) or **(1)** at 100 mg/kg **(F)** over AUC after 1^{st} gavage with V, which serves as internal control. Data are expressed as the mean AUC ratio ± SEM; n=7; ^{∗} p<0.05; ^{∗∗} p<0.01.

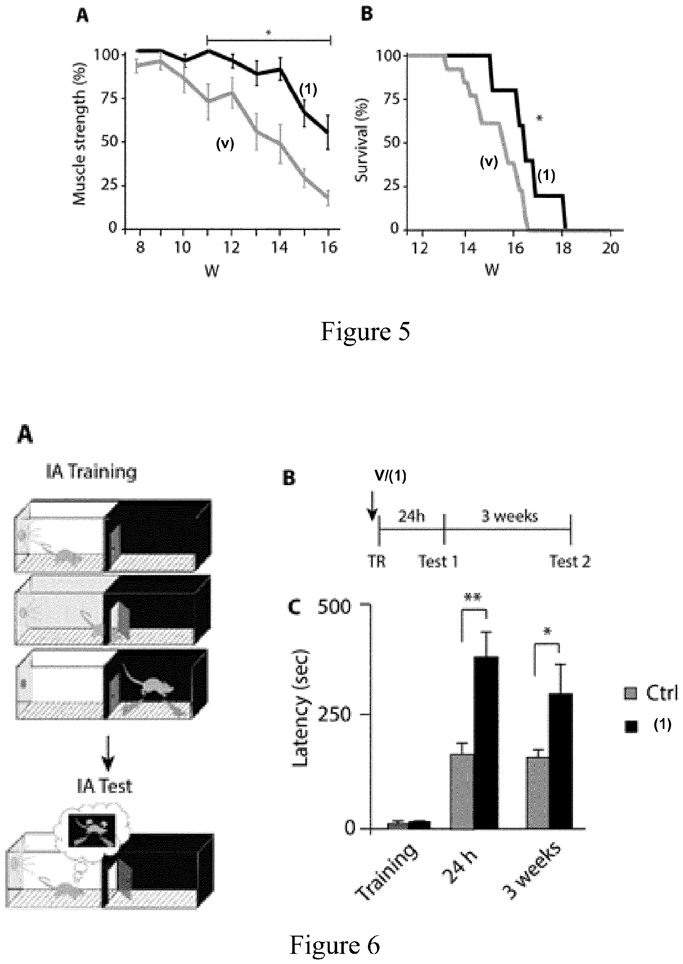

**Figure 5** shows the neuroprotective effect of compound of the invention **(1)** in the G93A SOD1 mouse model of ALS as described in Example 2. **A**: Grip strength test of G93A SOD1 male mice treated with Vehicle (V) or compound **(1)** (10 mg/kg) from postnatal day 30 to final stage (paralysis) measured by evolution of muscle strength every week (W) in the grip test procedure. Results are shown as percentage of maximal grip time, i.e. 5 minutes. Data are expressed as the average of the muscle strength percentage ± SEM; n=6; ^{∗} p<0.05; **B**: Survival percentage (%) of mice in weeks (W) treated from postnatal day 30 (weaning) to final stage (paralysis) with Vehicle alone or **(1)** (10mg/kg) as measured with Kaplan-Meier curves; n=6; * p<0.05.
**Figure 6** shows the cognitive effect of compound of the invention **(1)** in the mouse model of inhibitory avoidance (IA) as described in Example 2. **A**: Schematic representation of the task, including training (mild electrical footshock received in the dark compartment of an IA apparatus) and testing (latency of the mouse to go back to the dark compartment of an IA apparatus); **B**: Experimental schedule; **C**: Latencies of mice treated before training with Vehicle (V) or compound **(1)** (100 mg/kg) to enter the dark compartment of an IA apparatus at training, at Test 1 (24h after training) and at Test 2 (3 weeks after training). Data are shown as group average of latency values ± SEM; n=8-9; * p<0.05, ** p<0.01.

### Detailed description

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it such as a preventive early asymptomatic intervention; (b) inhibiting the disease, i.e., arresting its development; or relieving the disease, i.e., causing regression of the disease and/or its symptoms or conditions such as improvement or remediation of damage. In particular, the methods, uses, formulations and compositions according to the invention are useful for enhancing lactate, particular in the treatment of abnormal energy metabolism in the central nervous system.

The term "subject" as used herein refers to mammals. For examples, mammals contemplated by the present invention include human, primates, domesticated animals such as cattle, sheep, pigs, horses, laboratory rodents, other pets and the like.

The term "subject at risk of suffering from a disorder related to a deficiency in intracerebral energy metabolism or in the central nervous system (CNS)" refers to a subject presenting abnormal CNS energy metabolism, such as in neurological diseases.

The term "neurological disorder" according to the invention includes a neurodegenerative disease or a disorder characterized by a degeneration of the central nervous system, as found in amyotrophic lateral sclerosis (ALS), dementia - in particular Alzheimer's disease, frontotemporal dementia (FTD), dementia with Lewy bodies (LBD) -, Parkinson's disease, multiple sclerosis, stroke, traumatic brain injury or a psychotic disorder such as depression, schizophrenia, mild cognitive impairments and epilepsy. Subjects affected by those disorders which are presenting a deficiency energy metabolism in the central nervous system.

The term "effective amount" as used herein refers to an amount of at least one compound of the invention or a pharmaceutical formulation thereof according to the invention that elicits the biological or medicinal response in a tissue, system, animal or human that is being sought. In one embodiment, the effective amount is a "therapeutically effective amount" for the alleviation of the symptoms of the disease or condition being treated. In another embodiment, the effective amount is a "prophylactically effective amount" for prophylaxis of the symptoms of the disease or condition being prevented. The term also includes herein the amount of compound of the invention sufficient to reduce the progression of the disease, notably to reduce or inhibit the progression of a neurodegenerative disorder and thereby elicit the response being sought (i.e. an "effective amount").

The term "efficacy" of a treatment according to the invention can be measured based on changes in the course of disease in response to a use or a method according to the invention. For example, the efficacy of a treatment can be measured by an increase of lactate levels in the central nervous system, as well as by imaging techniques including Positron Emission Tomography (PET) with fluorine-18 (¹⁸F)-labeled 2-fluoro-2-deoxy-D-glucose as tracer or carbon-11, (¹¹C) Pittsburgh compound B (PIB), carbon-13 (¹³C), phosphorus-31 (³¹P), proton magnetic resonance spectroscopy (¹H) MRS to evaluate the bioenergetics status in the brain. Effective treatment is indicated by an increase in cognitive performance (e.g. memory, reasoning test), preservation of neuronal activity, which, in the case of motor neuron degeneration, can be measured by muscle activity, as well as clinical diagnosis relevant to a specific indication.

The term "alkyl" when used alone or in combination with other terms, comprises a straight chain or branched C₁-C₂₀ alkyl which refers to monovalent alkyl groups having 1 to 20 carbon atoms. This term is exemplified by groups such as methyl, ethyl, n-propyl, i-propyl, n-butyl, s-butyl, i-butyl, t-butyl, n-pentyl, 1-ethylpropyl, 2-methylbutyl, 3-methylbutyl, 2,2-dimethylpropyl, n-hexyl, 2-methylpentyl, 3-methylpentyl, 4-methylpentyl, n-heptyl, 2-methylhexyl, 3-methylhexyl, 4-methylhexyl, 5-methylhexyl, n-heptyl, n-octyl, n-nonyl, n-decyl, tetrahydrogeranyl, n-dodecyl, n-tridecyl, n-tetradecyl, n-pentadecyl, n-hexadecyl, n-octadecyl, n-nonadecyl, and n-eicosanyl and the like. Preferably, these include C₁-C₉ alkyl, more preferably C₁-C₆ alkyl, especially preferably C₁-C₄ alkyl, which, by analogy, refer respectively to monovalent alkyl groups having 1 to 9 carbon atoms, monovalent alkyl groups having 1 to 6 carbon atoms and monovalent alkyl groups having 1 to 4 carbon atoms. Particularly, those include C₁-C₆ alkyl.

The term "alkoxy" refers to the group -O-R where R includes "C₁-C₆ alkyl", "aryl", "heteroaryl", "aryl C₁-C₆ alkyl" or "heteroaryl C₁-C₆ alkyl". Preferred alkoxy groups include for example, methoxy, ethoxy, phenoxy and the like.

Unless otherwise constrained by the definition of the individual substituent, the term "substituted" refers to groups substituted with from 1 to 5 substituents selected from the group consisting of "C₁-C₆ alkyl," "C₃-C₈-cycloalkyl," "heterocycloalkyl," "C₁-C₆ alkyl aryl," "C₁-C₆ alkyl heteroaryl," "C₁-C₆ alkyl cycloalkyl," "C₁-C₆ alkyl heterocycloalkyl," "cycloalkyl C₁-C₆ alkyl," "heterocycloalkyl C₁-C₆ alkyl," "amino," "aminosulfonyl," "ammonium," "alkoxy," "acyl amino," "amino carbonyl," "aryl," "aryl C₁-C₆ alkyl," "heteroaryl," "heteroaryl C₁-C₆ alkyl," "sulfinyl," "sulfonyl," "sulphonamide", "alkoxy," "alkoxy carbonyl," "carbamate," "sulfanyl," "halogen," "carboxy," trihalomethyl, cyano, hydroxy, mercapto, nitro, trihalo methyloxy, trihalo methylthio and the like.

"Pharmaceutically active derivative" refers to any compound that upon administration to the recipient, is capable of providing directly or indirectly, the activity disclosed herein. The term "indirectly" also encompasses prodrugs which may be converted to the active form of the drug via endogenous enzymes or metabolism. The prodrug is a derivative of the compound according to the invention and presenting lactate enhancing activity that has a chemically or metabolically decomposable group, and a compound that may be converted into a pharmaceutically active compound *in vivo* under physiological conditions.

The prodrug is converted into a compound according to the present invention by a reaction with an enzyme, gastric acid or the like under a physiological condition in the living body, e.g. by oxidation, reduction, hydrolysis or the like, each of which is carried out enzymatically. These compounds can be produced from compounds of the present invention according to well-known methods. The term "indirectly" also encompasses metabolites of compounds according to the invention.

The term "metabolite" refers to all molecules derived from any of the compounds according to the present invention in a cell or organism, preferably mammal.

In the context of the present invention are encompassed pharmaceutically acceptable salts, complexes, hydrates, solvates, or polymorphs, tautomers, geometrical isomers, optically active forms and pharmaceutically active derivatives of compounds of the invention. Unless stated to the contrary, the present invention includes all such possible diastereomers as well as their racemic mixtures, their substantially pure resolved enantiomers, all possible geometric isomers, and pharmaceutically acceptable salts thereof. Mixtures of stereoisomers, as well as isolated specific stereoisomers, are also included. During the course of the synthetic procedures used to prepare such compounds, or in using racemization or epimerization procedures known to those skilled in the art, the products of such procedures can be a mixture of stereoisomers. Many organic compounds exist in optically active forms having the ability to rotate the plane of plane-polarized light. In describing an optically active compound, the prefixes D and L or R and S are used to denote the absolute configuration of the molecule about its chiral center(s).

In the context of the present invention, « pharmaceutically acceptable salt thereof » refers to salts which are formed from acid addition salts formed with an acid, said acid may be an inorganic acid (e.g. hydrochloric acid, hydrobromic acid, sulfuric acid, phosphoric acid, nitric acid, and the like), or an organic acid such as acetic acid, fumaric acid, oxalic acid, tartaric acid, succinic acid, malic acid, malonic acid, fumaric acid, maleic acid, ascorbic acid, lactic acid or benzoic acid.

Pharmaceutically acceptable salts for the instant disclosure may be selected among salts formed with acids such as hydrochloric acid.

The term "pharmaceutical formulation" refers to preparations which are in such a form as to permit biological activity of the active ingredient(s) to be unequivocally effective and which contain no additional component which would be toxic to subjects to which the said formulation would be administered.

### Compounds according to the invention

According to a particular aspect of the invention, are provided compounds of Formula (I): 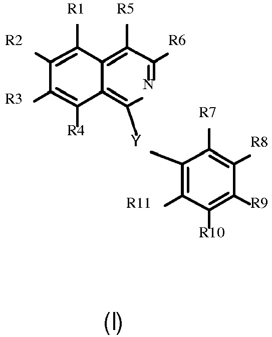 wherein R¹, R², R³, R⁴, R⁵ and R⁶ are independently selected from H, halogen, optionally substituted alkoxy, optionally substituted C₁-C₆ alkyl, optionally substituted amine, optionally substituted carboxylic acid or ester, nitro and nitrile; R⁷, R⁸, R⁹, R¹⁰ and R¹¹ independently selected from H, halogen, optionally substituted alkoxy, optionally substituted C₁-C₆ alkyl, optionally substituted amine, optionally substituted carboxylic acid or ester, nitro and nitrile and a group of Formula **(II):** -(X)ₘ-CR¹²R¹³R¹⁴ **(II)** wherein at least one group of R⁷, R⁸, R⁹, R¹⁰ and R¹¹ is a group of Formula **(II);** X is selected from O, NR¹⁵, S, CH₂ and hydrazine (-N-N-), m is an integer elected from 0 and 1 and R¹², R¹³ and R¹⁴ are independently selected from H, OH, optionally substituted alkoxy, optionally substituted C₁-C₆ alkyl and halogen, wherein at least one of R¹², R¹³ and R¹⁴ is F or Cl; Y is selected from - CR¹⁶R¹⁷- and -NR¹⁸-; R¹⁵ is selected from H, optionally substituted alkoxy and optionally substituted C₁-C₆ alkyl; R¹⁶ and R¹⁷ are independently selected from H, halogen, optionally substituted alkoxy, optionally substituted C₁-C₆ alkyl and optionally substituted aryl; R¹⁸ is independently selected from H or optionally substituted C₁-C₆ alkyl; any pharmaceutically acceptable salts, hydrates, solvates, or polymorphs, tautomers, geometrical isomers, optically active forms, enantiomeric mixtures thereof, and mixtures thereof, for the prevention, the repression or treatment of a disease or disorder related to a deficiency in energy metabolism in the central nervous system and/or a neurological disorder, in particular amyotrophic lateral sclerosis (ALS), dementia - in particular Alzheimer's disease, frontotemporal dementia (FTD), dementia with Lewy bodies (LBD) -, Parkinson's disease, multiple sclerosis, stroke, traumatic brain injury or a psychotic disorder such as depression, schizophrenia, mild cognitive impairments and epilepsy and for enhancing cognitive and memory functions.

According to another particular aspect of the invention, are provided compounds of Formula (I) wherein X is selected from O, NR¹⁵, S and hydrazine (-N-N-).

According to another particular aspect of the invention, are provided compounds of Formula (I) wherein R¹, R², R³, R⁴, R⁵ and R⁶ are independently selected from H, halogen, optionally substituted alkoxy such as methoxy, C₁-C₆ alkyl optionally substituted by halogen C₁-C₆ alkoxy, amino, nitro, optionally substituted amine, optionally substituted carboxylic acid or ester, nitro (NO₂) and nitrile.

In a more particular embodiment, is provided a compound according to Formula (I), wherein Y is -NR¹⁸.

In another more particular embodiment, is provided a compound according to Formula (I), wherein Y is -CR¹⁶R¹⁷.

In another more particular embodiment, is provided a compound according to Formula (I), wherein Y is -CR¹⁶ R¹⁷ and when one of R¹⁶ and R¹⁷ is a halogen, the other is not a halogen. In a more particular embodiment, is provided a compound according to Formula (I), wherein R¹⁸ is H.

In another more particular embodiment, is provided a compound according to Formula (I), wherein CR¹⁶ and R¹⁷ are H.

In another more particular embodiment, is provided a compound according to Formula (I), wherein R⁵ and R⁶ are H.

In another more particular embodiment, is provided a compound according to Formula (I), wherein R¹ and R⁴ are H.

In another more particular embodiment, is provided a compound according to Formula (I), wherein R² and R³ are independently selected from H and optionally substituted alkoxy such as methoxy.

In another more particular embodiment, is provided a compound according to Formula (I), wherein R¹, R², R³ and R⁴ are H.

In another more particular embodiment, is provided a compound according to Formula (I), wherein R¹ to R⁶ are H.

In another more particular embodiment, is provided a compound according to Formula (I), wherein R¹, R⁴ and R⁶ are H.

In another more particular embodiment, is provided a compound according to Formula (I), wherein R⁹ is a group-(X)ₘ-CR¹²R¹³R¹⁴.

In another more particular embodiment, is provided a compound according to Formula (I), wherein R⁹ is a group-(X)ₘ-CR¹²R¹³R¹⁴ and R⁷, R⁸, R¹⁰ and R¹¹ are H.

In another more particular embodiment, is provided a compound according to Formula (I), wherein m is 1.

In another more particular embodiment, is provided a compound according to Formula (I), wherein m is 0.

In another more particular embodiment, is provided a compound according to Formula (I), wherein X is O.

In another more particular embodiment, is provided a compound according to Formula (I), wherein R¹², R¹³ and R¹⁴ are F.

In another more particular embodiment, is provided a compound according to Formula (I), wherein R⁹ is OCF₃.

In another more particular embodiment, is provided a compound according to Formula (I), wherein R⁹ is CF₃.

In another more particular embodiment, is provided a compound according to Formula (I) with the proviso that it is not 1-[[4-(trifluoromethoxy)phenyl]methyl]isoquinoline.

In a more particular embodiment, compounds of the invention are selected from the following group:
6,7-dimethoxy-N-(4-(trifluoromethoxy)phenyl)isoquinolin-1-amine;
6,7-dimethoxy-N-(4-(trifluoromethyl)phenyl)isoquinolin-1-amine;
N-(4-(trifluoromethoxy)phenyl)isoquinolin-1-amine; and
6,7-dimethoxy-1-(4-(trifluoromethoxy)benzyl)isoquinoline; any pharmaceutically acceptable salts, complexes, hydrates, solvates, or polymorphs, tautomers, geometrical isomers, optically active forms and pharmaceutically active.

According to a particular aspect of the invention are provided compounds of the invention and their pharmaceutically acceptable salts selected among salts formed with hydrochloric acid or hydrobromic acid, sulfuric acid, phosphoric acid, nitric acid, and the like, or an organic acid such as acetic acid, fumaric acid, oxalic acid, tartaric acid, succinic acid, malic acid, malonic acid, fumaric acid, maleic acid, ascorbic acid, lactic acid or benzoic acid.

Among their salts mention may be made especially of the salts selected from the following list:
6,7-dimethoxy-N-(4-(trifluoromethoxy)phenyl)isoquinolin-1-amine hydrochloride;
6,7-dimethoxy-N-(4-(trifluoromethyl)phenyl)isoquinolin-1-amine hydrochloride;
N-(4-(trifluoromethoxy)phenyl)isoquinolin-1-amine hydrochloride; and
6,7-dimethoxy-1-(4-(trifluoromethoxy)benzyl)isoquinoline hydrochloride.

According to a particular aspect of the invention, is provided a pharmaceutical composition comprising at least one compound of Formula (I) ^{∗} as defined herein and a pharmaceutically acceptable carrier, diluent or excipient thereof wherein said at least one compound is not a compound selected from the following list:
N-[2- (trifluoromethyl)phenyl]isoquinolin- 1- amine;
N-[3- (trifluoromethyl)phenyl]isoquinolin- 1- amine; and
N-[4- (trifluoromethyl)phenyl]isoquinolin- 1- amine;
*wherein R¹, R², R³, R⁴, R⁵ and R⁶ are independently selected from H, halogen, C₁-C₆ alkyl, optionally substituted C₁-C₆ alkoxy, optionally substituted amine, nitro and nitrile, wherein C₁-C₆alkoxy is optionally substituted by halogen; R⁷, R⁸, R⁹, R¹⁰ and R¹¹ are as defined in any of the preceding claims.

The compounds of invention have been named according the IUPAC standards used in the ChemDraw (product version ultra 13.0).

According to another aspect the invention provides a process for the preparation of a compound according to Formula (I) comprising the step of reacting an aniline intermediate of Formula **(iii)** with an intermediate of Formula **(ii)** wherein Z is selected from Iodine, Bromide or O-triflate in a polar solvent to form a compound of Formula (Ia):

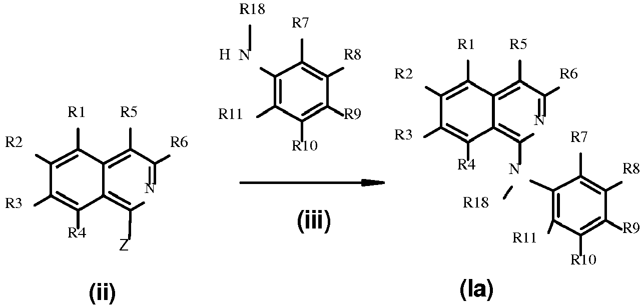

According to another aspect, is provided a process for the preparation of a compound according to Formula (I), comprising the step of reacting a compound of Formula (Ia) wherein R¹⁸ is H to lead to a compound of Formula (I) wherein R¹⁸ is an optionally substituted C₁-C₆ alkyl (e.g. by N- alkylation).

According to another aspect, is provided a process for the preparation of a compound according to Formula (I) comprising a reduction step of a carbonyl intermediate of Formula (viii) (e.g. by NaBH₃CN in the presence of ZnCl₂ or by catalytic hydrogenation (H₂ in the presence of Pd/C and acid trace) to lead to a compound of Formula (Iab):

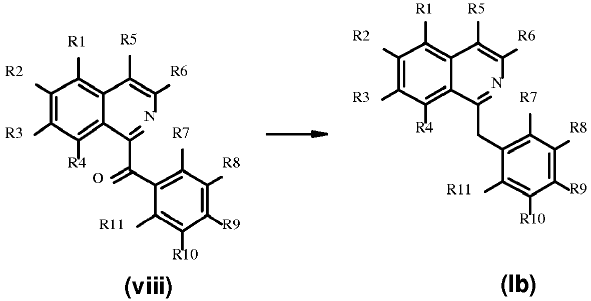

According to another aspect, is provided a process for the preparation of a compound according to Formula (I) wherein the carbonyl group of an intermediate **(viii)** is reduced to lead to a compound of Formula (I) wherein at least one of the groups R¹⁶ and R¹⁷ is a halogen, an optionally substituted optionally substituted alkoxy, an optionally substituted C₁-C₆ alkyl, or an optionally substituted aryl (e.g. through a Grignard reaction or reacting an intermediate **(viii)** with (Diethylamino)sulfur trifluoride).

### Compositions according to the invention

The invention provides pharmaceutical or therapeutic agents as compositions for their use in treating a subject, preferably a mammalian subject, and most preferably a human patient who is suffering from a medical disorder, and in particular a neurological disorder, including amyotrophic lateral sclerosis (ALS), dementia - in particular Alzheimer's disease, frontotemporal dementia (FTD), dementia with Lewy bodies (LBD) -, mild cognitive impairments, Parkinson's disease, multiple sclerosis, depression, schizophrenia, stroke, traumatic brain injury or epilepsy.

Agent of the invention or formulations thereof may be administered as a pharmaceutical formulation, which can contain one or more agents according to the invention in any form described herein. The compositions according to the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use by injection or continuous infusion. Injectable compositions are typically based upon injectable sterile saline or phosphatebuffered saline or other injectable carriers known in the art. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

Compositions of this invention may be liquid formulations including, but not limited to aqueous or oily suspensions, solutions, emulsions, syrups, and elixirs. The compositions may also be formulated as a dry product for reconstitution with water or other suitable vehicle before use. Such liquid preparations may contain additives including, but not limited to, suspending agents, emulsifying agents, non-aqueous vehicles and preservatives. Suspending agents include, but are not limited to, sorbitol syrup, methylcellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Preservatives include, but are not limited to, methyl or propyl p-hydroxybenzoate and sorbic acid. Dispersing or wetting agents include but are not limited to poly(ethylene glycol), glycerol, bovine serum albumin, Tween^{®}, Span^{®}.

Compositions of this invention may also be formulated as a depot preparation, which may be administered by implantation or by intramuscular injection.

Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maize starch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art.

The compounds of this invention can also be administered in sustained release forms or from sustained release drug delivery systems.

According to a particular embodiment, compositions according to the invention are for intravenous use.

According to a particular aspect, the formulations of the invention are oral formulations.

In another particular aspect, the compositions according to the invention are adapted for delivery by repeated administration.

According to a particular embodiment, compositions of the invention are veterinary compositions.

Further materials as well as formulation processing techniques and the like are set out in Part 5 of Remington's "The Science and Practice of Pharmacy", 22nd Edition, 2012, University of the Sciences in Philadelphia, Lippincott Williams & Wilkins*.*

### Mode of administration

Compounds and formulations thereof according to this invention may be administered in any manner including orally, parenterally, intravenously, intrathecally, rectally, or combinations thereof. Compounds and formulations thereof according to this invention may be also administered by inhalation or intradermally. Parenteral administration includes, but is not limited to, intravenous, intra-arterial, intra-peritoneal, subcutaneous and intramuscular. The compositions of this invention may also be administered in the form of an implant, which allows slow release of the compositions as well as a slow controlled i.v. infusion.

### Combination

According to the invention, compounds and pharmaceutical formulations thereof can be administered alone or in combination with a co-agent useful for treating, and/or stabilizing a neurological disorder such as amyotrophic lateral sclerosis (ALS), dementia - in particular Alzheimer's disease, frontotemporal dementia (FTD), dementia with Lewy bodies (LBD) -, mild cognitive impairments, Parkinson's disease, multiple sclerosis, depression, schizophrenia, stroke, traumatic brain injury or epilepsy.

The disclosure encompasses the administration of a compound of the invention or a formulation thereof wherein it is administered to a subject prior to, simultaneously or sequentially with other therapeutic regimens or co-agents useful for treating, and/or stabilizing a neurodegenerative disorder.

The disclosure encompasses the administration of a compound of the invention or a formulation thereof wherein it is administered to a subject prior to, simultaneously or sequentially with other therapeutic regimens or co-agents useful for the prevention and/or treatment of psychiatric disorders or improving cognitive and memory functions.

Examples of co-agents useful in combination with compounds of the invention and pharmaceutical formulations thereof include drug therapies useful for the treatment of the cognitive symptoms (memory loss, confusion, and problems with thinking and reasoning) of Alzheimer's disease such as cholinesterase inhibitors and memantine. Non-limiting examples of cholinesterase inhibitors include donepezil, rivastigmine and galantamine.

A co-agent according to the invention may include donepezil and memantine in a single dosage form.

Examples of co-agents useful in combination with compounds of the invention and pharmaceutical formulations thereof include drug therapies useful to treat Amyotrophic Lateral Sclerosis such as riluzole and ederavone.

Examples of co-agents useful in combination with compounds of the invention include medications for behavioural changes, which act as adjunct treatments but which do not directly treat the symptoms of Alzheimer's disease, such as one or more of antidepressants, anxiolytics or antipsychotic medications. Non-limiting examples of suitable antidepressants include citalopram, fluoxetine,
paroxeine, sertraline, trazodone and esketamine. Non-limiting examples of suitable anxiolytics include lorazepam and oxazepam. Non-limiting examples of suitable antipsychotic medications include aripiprazole, clozapine, haloperidol, olanzapine, quetiapine, risperidone and ziprasidone.

A compound of the invention or a formulation thereof according to the invention that is administered simultaneously with said co-agents can be administered in the same or different composition(s) and by the same or different route(s) of administration.

According to one embodiment, is provided a pharmaceutical formulation comprising a compound of the invention combined with at least one co-agent useful for treating, and/or stabilizing, a neurodegenerative disorder and at least one pharmaceutically acceptable carrier. Other combinations will be readily appreciated and understood by persons skilled in the art. In some embodiments, the compounds of the invention can be used to attenuate or reverse the activity of a drug suitable for treatment of a neurological disease as described herein, and/or limit the adverse effects of such drugs.

As persons skilled in the art will readily understand, the combination can include the therapeutic agents and/or a pharmaceutical composition comprising same, according to at least some embodiments of the invention and one other drug; the therapeutic agents and/or a pharmaceutical composition comprising same, as recited herein, with two other drugs, the therapeutic agents and/or a pharmaceutical composition comprising same, as recited herein, with three other drugs, etc. The determination of the optimal combination and dosages can be determined and optimized using methods well known in the art.

The therapeutic agent according to the present invention and one or more other therapeutic agents can be administered in either order or simultaneously.

### Methods according to the invention

According to another aspect, the disclosure provides a method of preventing or treating a disorder related to a deficiency in energy metabolism in the central nervous system.

According to another aspect, the disclosure provides a method of preventing and/or treating a neurodegenerative disorder.

According to another aspect, the disclosure provides a method of preventing and/or treating a psychotic disorder.

According to another aspect, the disclosure provides a method of increasing the lactate secretion by astrocytes.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

In another embodiment, the invention provides a pharmaceutical composition containing at least one compound of the invention and a pharmaceutically acceptable carrier, diluent or excipient thereof.

Compounds according to the present invention comprise a compound according to Formula (I), its tautomers, its geometrical isomers, its optically active forms as enantiomers, diastereomers and its racemate forms, as well as pharmaceutically acceptable salts thereof. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description. Such modifications are intended to fall within the scope of the appended claims, which define the scope of the invention.

The invention having been described, the following examples are presented by way of illustration, and not limitation.

### Synthesis of compounds of the invention

The novel derivatives according to Formula (I) can be prepared from readily available starting materials using the following general methods and procedures. It will be appreciated that where typical or preferred experimental conditions (i.e. reaction temperatures, time, moles of reagents, solvents etc.) are given, other experimental conditions can also be used unless otherwise stated. Optimum reaction conditions may vary with the particular reactants or solvents used, but such conditions can be determined by the person skilled in the art, using routine optimisation procedures.

The general synthetic approaches for obtaining compounds of Formula (I) are depicted in Schemes 1 and 2 below.

Compounds of Formula (I), wherein Y is NH (Formula Ia) can be synthesized as described in Scheme 1 below. An isoquinoline intermediate of Formula **(i)** wherein Z can be Iodine, Bromide or O-triflate is reacted with an acid such as trifluoroacetic acid or an alkyl sulfonic acid derivative (e.g. methyl sulfonic acid or trifluoromethylsulfonic acid) in a polar solvent such as isopropanol to form an intermediate of Formula **(ii)**.

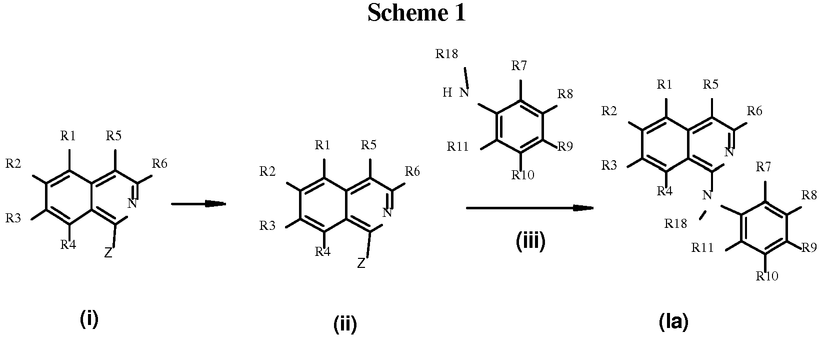

Then, an aniline intermediate of Formula **(iii)** is added to the intermediate of Formula (ii) in a polar solvent such as tert-butanol and the mixture is let reacted under reflux overnight and then after cooling, hydrolysed in presence of a base such as with a saturated NaHCO₃, Na₂CO₃, KHCO₃, K₂CO₃, NaOH or diluted KOH solution. After extraction, e.g. with dichloromethane drying and evaporation under reduced pressure, the crude is separated on silica gel to lead to a compound of Formula (Ia).

Compounds of Formula **(I)**, wherein Y is CH₂ (Formula Ib) can be synthesized as described in Scheme 2 below.

An ethylamine intermediate of Formula **(iv)** is mixed with a phenyl acetic acid intermediate or a phenylacetyl chloride intermediate of Formula **(v)** and the mixture heated, e.g. at about 180°C for about 2h. Then after cooling, dichloromethane is added and the organic layer is washed by water. After extraction, e.g. with dichloromethane drying and evaporation under reduced pressure, intermediate amide compound of Formula **(vi)** is obtained. Compound of Formula **(vi)** is dissolved in a polar solvent such as toluene under heating, e.g. 90°C and a solution of POCl₃ or P₂O₅ is added drop to drop and let reacted under heating, e.g. at about 100°C for about 2 h. After cooling to room temperature and partial elimination of the solvent, warm water (e.g. 60°C) is carefully added before stirring until complete solubilization.

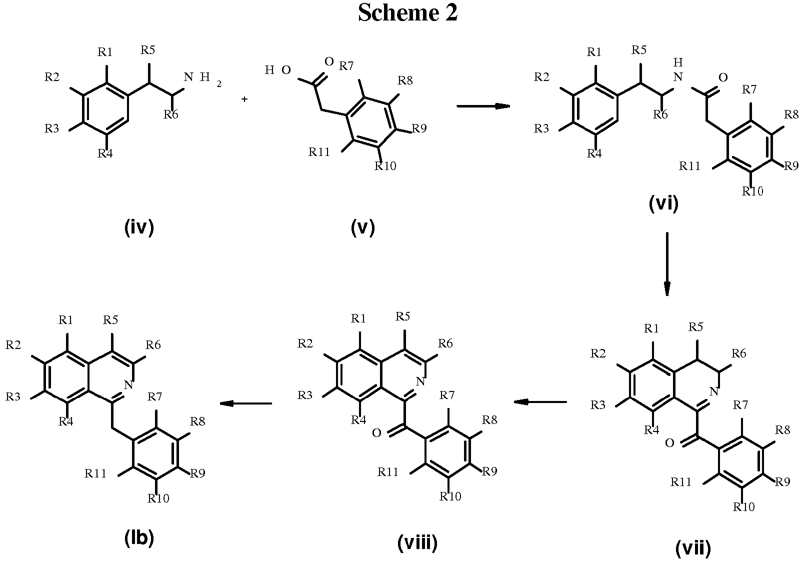

The aqueous phase was alkalized, e.g. with 20% NaOH, then extracted with an apolar solvent such as CH₂Cl₂ and dried on Magnesium sulfate. After elimination of solvent under reduce pressure, a dihydroisoquinoleine intermediate of Formula **(vii)** is obtained. MnO₂ and Na₂SO₄ are added to the dihydroisoquinoleine intermediate of Formula **(vii)** in a polar solvent such as toluene. The whole mixture is heated for about 2h at reflux. After cooling and filtration, the crude is separated on silica gel to lead to a carbonyl intermediate of Formula **(viii)** or, alternatively, a dihydroisoquinoleine intermediate of Formula **(vii)** is subjected to heating in presence of sulfur or Pd/C to lead to a carbonyl intermediate of Formula **(viii).** This carbonyl intermediate is then reduced in glycol ethylene for example by the addition of hydrazine monohydrate or in presence of a hydride such as NaBH₃CN in presence de ZnCl₂ or by catalytic hydrogenation (H₂ in presence of Pd/C in presence of acid) and whole mixture was heated for about 30 min at about 120°C. After, KOH in glycol ethylene is added and the whole mixture is heated for about 3 h at about 190°C. After cooling and extraction with an apolar solvent such as dichloromethane, the organic layer is washed successively by water and bride. After drying and evaporation under reduce pressure, the crude is separated on silica gel under nitrogen to lead to a compound of Formula (Iab).

According to one aspect, a compound according to Formula (I), wherein groups at least one group of R¹⁶ to R¹⁸ is different from H can be obtained N- alkylation of an intermediate of Formula (Ia) or by reduction of a carbonyl intermediate of Formula **(vii)** as described herein.

### Patients

In an embodiment, patients according to the invention are subjects suffering from a disorder related to a deficiency in energy metabolism in the central nervous system.

In a particular embodiment, patients according to the invention are suffering from a neurodegenerative disorder.

In a particular embodiment, patients according to the invention are suffering from a psychotic disorder.

In an embodiment, patients according to the invention are patients that are at risk of suffering from a disorder related to a deficiency in energy metabolism in the central nervous system such as subjects genetically pre-disposed in suffering from amyotrophic lateral sclerosis (ALS) or a dementia, including Alzheimer's diseases, frontotemporal dementia (FTD), dementia with Lewy bodies (LBD) or subjects genetically pre-disposed in suffering from depression or subjects genetically pre-disposed in suffering from a disorder selected from mild cognitive impairments, Parkinson's disease, multiple sclerosis, schizophrenia, stroke, traumatic brain injury and epilepsy.

According to a particular aspect, compounds and methods of the invention are useful for the prevention and/or treatment of a neurodegenerative disorder.

According to a further aspect, a neurodegenerative disorder is amyotrophic lateral sclerosis (ALS).

According to another particular aspect, compounds and methods of the invention are useful for the prevention and/or treatment of a psychiatric disorder.

According to another further aspect, a psychiatric disorder is depression.

According to another particular aspect, patients are suffering from mild cognitive impairments due to ageing such as age-associated cognitive decline and age-related memory impairments. According to another particular aspect, methods of the invention are useful for enhancing cognitive and memory functions in healthy subjects. The present invention is not to be limited in scope by the specific embodiments and drawings described herein, which are intended as single illustrations of individual aspects of the invention, and functionally equivalent methods and components are within the scope of the invention. The examples illustrating the invention are not intended to limit the scope of the invention in any way.

### EXAMPLES

The following studies are conducted to support the effectiveness of compounds of the invention according to the invention.

### Example 1: Synthesis of compounds of the invention

All synthetic reagents and solvents are used as is. If necessary, the solvents used in the reactions are previously dried and/or distilled in accordance with the state of the art. Some solvents are commercially available in an anhydrous state and are used as is.

### Reactive Conditions

When anhydrous conditions are required, the glassware is first dried in an oven (T>100°C). All reactions are performed under nitrogen atmosphere. The ambient temperature (rt) refers to 20-25°C. The reaction temperature of -78°C is obtained by freezing a water bath of acetone with carboglace or liquid nitrogen. The temperature of 0°C corresponds to the use of an ice water bath. For heating, an oil bath with a temperature sensor is used for temperature control. The progress of the reactions is controlled by thin layer chromatography (CCM). These CCM (DC Kieselgel 60 F254, UV254 plates) correspond to aluminium plates precoated with silica gel and UV fluorescent indicator. The plates are revealed under UV light (254 and 365 nm) or using a developer adapted to the molecules to be visualized. A phosphomolybdic acid solution is commonly used as an oxidizing developer with or without thermal revelation.

### Purification Techniques

### Flash chromatography:

Silica gel (Kieselgel 60 from MN, 15-40 µm from Macherey-Nagel) is used in the purification of raw products by Flash chromatography. The samples are either deposited directly at the column head or applied as a solution in a silica gel suspension.

### Automated chromatography flash:

The purification system used is a Combiflash Companion ^{™} from Teledyne Isco. The raw samples are dissolved in a small amount of suitable solvent and applied to pre-conditioned RediSep^{®} columns. These columns are placed in the Combiflash Companion purification system ^{™} and purification is performed using a solvent gradient program. The system is used with an automated collector. The detection is carried out by UV or by the collection of all the fractions analysed by HPLC.

### Nuclear magnetic resonance (NMR) spectrometry:

NMR spectra are recorded using a Bruker UltraShield spectrometer operating at 400 MHz (1H) and 100 MHz (13C). Spectrum calibration is performed by adding tetramethyl silane (TMS) to the deuterated solvent as the internal reference. The calibration is obtained by setting the 0 to the TMS signal.

For fluorine 19, CFC13 is used as an external reference. Chemical displacements are reported in parts per million (ppm) and coupling constants are given in Hertz (Hz). Abbreviations for the multiplicity of proton and carbon signals are: s singlet, d doublet, dd doublet of doublet, dt doublet of triplets, ddt doublet of triplets, t triplet, tt triplet of triplets, q, quintet, m multiplet.

### Mass spectrometry (SM):

Mass spectra are performed using a Bruker Q-TOF maXis coupled to a Dionex Ultimate 3000 RSLC chain used in FIA (Flow Injection Analysis = without column) with an ACN/H2O+0.1% formic acid mixture 65/35 to 200 µL/min as the solvent. The injection volume is 0.2 µL. Most of the time, the analyses are performed in positive mode with the ESI source (Electrospray Ionisation).

### Preparation of samples:

The samples are taken at a concentration of about 1 mg/mL with the solvent then diluted approximately 500 times (≈ 2 ng/µL) in methanol (sometimes another solvent more appropriate according to the structure of the compound to be analyzed: water, acetonitrile...). If the signal obtained is insufficient, the sample concentration is increased.

### Melting point measurement:

Melting points are measured using a STUART SMP3.

### High Performance Liquid Chromatography (HPLC):

HPLC analyses are performed on a Waters analytical HPLC system (Waters Delta 600 Multisolvent pump, Waters 600 system controller, Rheodyne 7725i injector with a 20 µl sample loop) controlled with Empower software and equipped with appropriate analytical column. The detection is carried out with a UV detector with photodiode strip (Waters 2996) and/or a refractometer.

Compounds 1 to 3 from **Table 1** below of Formula (I), in particular (Ia) were synthesized according the Scheme 1 and compound 4 of Formula (I), in particular (Ib), according to Scheme 2.

**Table 1**

| Compound | Y | R¹ | R² | R³ | R⁴ | R⁵ | R⁶ | R⁷ | R⁸ | R⁹ | R¹⁰ | R¹¹ |
|---|---|---|---|---|---|---|---|---|---|---|---|---|
| **1** | NH | H | OCH₃ | OCH₃ | H | H | H | H | H | OCF₃ | H | H |
| **2** | NH | H | OCH₃ | OCH₃ | H | H | H | H | H | CF₃ | H | H |
| **3** | NH | H | H | H | H | H | H | H | H | OCF₃ | H | H |
| **4** | CH₂ | H | OCH₃ | OCH₃ | H | H | H | H | H | OCF₃ | H | H |

### a) Preparation of 6,7-dimethoxy-N-(4-(trifluoromethoxy)phenyl)isoquinolin-1-amine (1)

Compound (1) was prepared as detailed in Scheme 3 below.

### a. Iodized salt formation

Trifluoroacetic acid (34 µl) is added dropwise to an isoquinoline **(ia)** (@rtMolecules) (m=120 mg, 0.38 mmol) in isopropanol (2 ml). The whole is drawn under vacuum one night to lead to salt intermediate **(iia).**

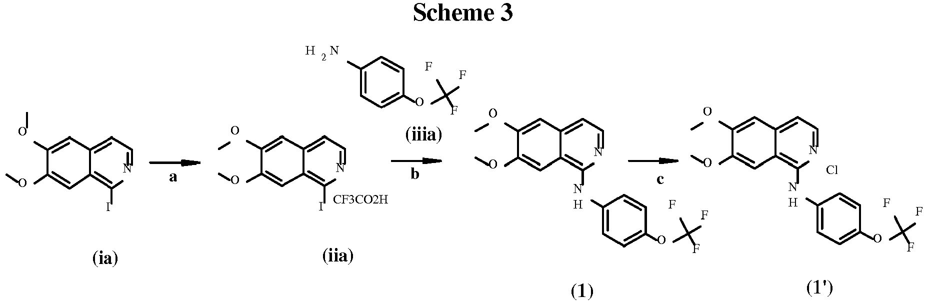

### b. Introduction of aniline to form compound (1)

To salt intermediate **(iia)** (m=61 mg, 0.19 mmol) in tert-butanol (v=1.4 ml), 4-trifluoromethoxyaniline (m=86 mg, 0.47 mmol) is added as intermediate **(iiia)** (AK Scientific (USA). The whole is refluxed overnight, then after cooling, hydrolysed with a saturated NaHCO₃ solution. After extraction with dichloromethane drying and evaporation, the crude is separated on silica gel (Eluant CH₂Cl₂/MeOH:97/3) to lead to compound of the invention **(1)** (m=55 mg, 79%).

### c. Formation of hydrochloride (1')

Compound **(1)** (m=55 mg, 0.16 mmol) is solubilized at 0°C in an AcOEt/EtOH mixture (16/2, 5 ml) and HCl (2M, in ethyl acetate, 0.3 ml) added. After 15 minutes agitation, solvent is removed under reduced pressure to obtain compound of the invention **(1')** which was characterized as follows: ¹H NMR (400 MHz, DMSO) δ(ppm) 12.67 (s, 1H), 11.37 (s, 1H), 8.35 (s, 1H), 7.71 (d, *J* = 8.9 Hz, 2H), 7.59 - 7.44 (m, 4H), 7.32 (d, *J* = 6.8 Hz, 1H), 4.01 (s, 3H), 3.99 (s, 3H). ¹³C NMR (101 MHz, DMSO) δ(ppm) 155.53, 151.04, 149.93, 135.67, 134.81, 127.93, 123.18, 121.84, 113.00, 112.97, 107.73, 105.96, 57.20, 56.73. DEPT 135 NMR (101 MHz, DMSO) δ 127.94, 123.19, 113.00, 107.73, 105.95, 57.20, 56.73.

### b) Preparation of 6,7-dimethoxy-N-(4-(trifluoromethyl)phenyl)isoquinolin-1-amine (2)

Compound (2) was prepared as detailed in Scheme 4 below.

### a. Iodized salt formation

The same protocol was used to obtain to salt intermediate **(iia)** as described above using the following amount: trifluoroacetic acid (71 µl) and isoquinoline **(ia)** (m=250 mg, 0.793 mmol) in isopropanol (4.2 ml).

### b. Introduction of aniline to form compound (2)

To salt intermediate **(iia)** (0.793 mmol) in tert-butanol (v=8 ml), 4-trifluoromethylaniline (m=320 mg, 1.98 mmol) is added as intermediate **(iiib).** The whole is refluxed overnight, then after cooling, the precipitate was solubilized in CH₂Cl₂ (250 ml)/ NaHCO₃ saturated aqueous solution (250 ml) mixture. After extraction with dichloromethane (3X) and washing with NaCl saturated aqueous solution, the organic phase was drying before evaporation under reduce pressure. The crude is separated on silica gel (Eluant CH₂Cl₂/MeOH: 99/1) to lead to the compound of the invention **(2)** (m=161 mg, 58.2%).

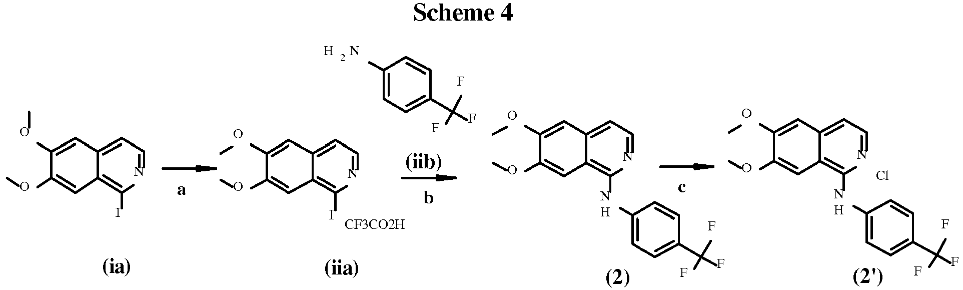

### b. Introduction of aniline to form compound (2)

To salt intermediate **(iia)** (0.793 mmol) in tert-butanol (v=8 ml), 4-trifluoromethylaniline (m=320 mg, 1.98 mmol) is added as intermediate **(iiib).** The whole is refluxed overnight, then after cooling, the precipitate was solubilized in CH₂Cl₂ (250 ml)/ NaHCO₃ saturated aqueous solution (250 ml) mixture. After extraction with dichloromethane (3X) and washing with NaCl saturated aqueous solution, the organic phase was drying before evaporation under reduce pressure. The crude is separated on silica gel (Eluant CH₂Cl₂/MeOH: 99/1) to lead to the compound of the invention **(2)** (m=161 mg, 58.2%).

### c. Formation of hydrochloride (2')

Compound **(2)** (m=161 mg, 0.462 mmol) is solubilized at 0°C in an AcOEt (3.2 ml) and HCl (0.4M in ethyl acetate, 1.2 ml) added. After 15 minutes agitation, solvent is removed under reduced pressure to obtain compound of the invention **(2')** which was characterized as follows: ¹H NMR (400 MHz, DMSO) δ(ppm) 13.04 (s, 1H), 11.91 (s, 1H), 8.53 (s, 1H), 7.91 (d, *J* = 8.5 Hz, 2H), 7.81 (d, *J* = 8.4 Hz, 2H), 7.57 (t, *J* = 3.4 Hz, 2H), 7.39 (d, *J* = 6.8 Hz, 1H), 4.04 (s, 3H), 4.00 (s, 3H). ¹³C NMR (101 MHz, DMSO) δ(ppm) 155.82, 151.15, 149.27, 140.82, 135.22, 127.54, 127.50, 127.31, 125.64, 113.68, 113.48, 107.69, 106.57, 57.38, 56.78. DEPT 135 NMR (101 MHz, DMSO) δ(ppm) 127.54, 127.50, 127.32, 125.64, 113.68, 107.69, 106.57, 57.38, 56.78.

### c) Preparation of N-(4-(trifluoromethoxy)phenyl)isoquinolin-1-amine (3)

Compound (3) was prepared as detailed in Scheme 5 below:

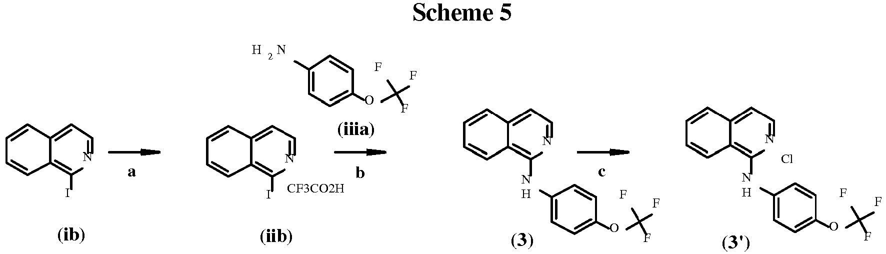

### a. Iodized salt formation

Trifluoroacetic acid (0.46 ml) is added dropwise to an isoquinoline **(ib)** (TCI Europe) (m=1.3g, 5.11 mmol) in isopropanol (27 ml). The whole is drawn under *vacuum* one night to lead to intermediate **(iib).**

### b. Introduction of aniline to form compound (3)

To salt intermediate **(iib)** (5.11 mmol) in tert-butanol (v=50 ml), 4-trifluoromethoxyaniline (AK Scientific (USA)) (1.73 ml, 10.22 mmol) is added as intermediate **(iiia)**. The whole is refluxed overnight, then after cooling, the precipitate was solubilized in CH₂Cl₂ (250 ml)/ NaHCO₃ saturated aqueous solution (250 ml) mixture. After extraction with dichloromethane (3X) and washing with NaCl saturated aqueous solution, the organic phase was drying before evaporation under reduce pressure. The crude is separated on silica gel (Eluant EP/AcOEt: 5/95) to lead to compound of the invention **(3)** (m=1.2g, 64%).

### c. Formation of hydrochloride (3')

Compound (3) (m=200 mg, 0.657 mmol) is solubilized at 0°C in an AcOEt/EtOH mixture (4.6 ml) and HCl (0.4M, in ethyl acetate, 1.7 ml) added. After 15 minutes agitation, solvent is removed under reduced pressure to obtain compound of the invention **(3')** which was characterized as follows: ¹H NMR (400 MHz, DMSO) δ(ppm) 12.81 (s, 1H), 11.70 (s, 1H), 9.04 (d, *J* = 8.2 Hz, 1H), 8.23 - 7.92 (m, 2H), 7.92 - 7.81 (m, 1H), 7.76 (d, *J* = 8.5 Hz, 2H), 7.64 (d, *J* = 6.6 Hz, 1H), 7.57 (d, *J* = 8.2 Hz, 2H), 7.41 (d, *J* = 6.7 Hz, 1H). ¹³C NMR (101 MHz, DMSO) δ(ppm) 152.03, 137.71, 135.41, 134.89, 129.12, 128.11, 126.32, 123.13, 119.01, 113.43, 67.48, 25.59. DEPT 135 NMR (101 MHz, DMSO) δ(ppm) 134.88, 129.12, 128.11, 126.32, 123.14, 113.43, 67.48, 25.59.

### d) Preparation of 6,7-dimethoxy-1-(4-(trifluoromethoxy)benzyl)isoquinoline (4)

Compound (5) was prepared as detailed in Scheme 6 below.

### a. Amide formation

2-(3,4-dimethoxyphenyl)ethanamine intermediate of Formula **(iva)** (TCI Europe) (m= 2 g, 11 mmol) and 2-(4-(trifluoromethoxy)phenyl)acetic acid intermediate of Formula **(va)** (Fluorochem (UK) (m= 2.4 g, 11 mmol) are heated at 180°C until 2h. Then after cooling, dichloromethane was added and the organic layer is washed by water. After drying and evaporation under reduce pressure, intermediate amide compound of Formula **(via)** was obtain (m=900 mg -21 %).

### b. Bishler Napieralski (BN) reaction

To intermediate amide compound of Formula **(via)** (m=87 0 mg, 2.27 mmol) in toluene (v=15 ml) at 90°C, a solution of POCl₃ (1.5 ml) was added drop to drop. The whole was heated 2h at 100°C. After cooling to room temperature and partial elimination of toluene, warm (60°C) water (30 ml) was carefully added before stirring until complete solubilization. The aqueous phase was alkalized with 20% NaOH then extracted with CH₂Cl₂ and dried on Magnesium sulfate. After elimination of solvent under reduce pressure, a dihydroisoquinoline intermediate of Formula **(viia)** is obtained (m=750 mg - 90 %) characterized as follows: ¹H NMR (400 MHz, CDC13) δ(ppm) 7.33 (d, J = 8.4 Hz, 2H), 7.13 (d, J = 8.5 Hz, 2H), 6.91 (s, 1H), 6.69 (s, 1H), 4.08 (s, 2H), 3.90 (s, 3H), 3.78 - 3.67 (m, 5H), 2.73 - 2.60 (m, 2H).

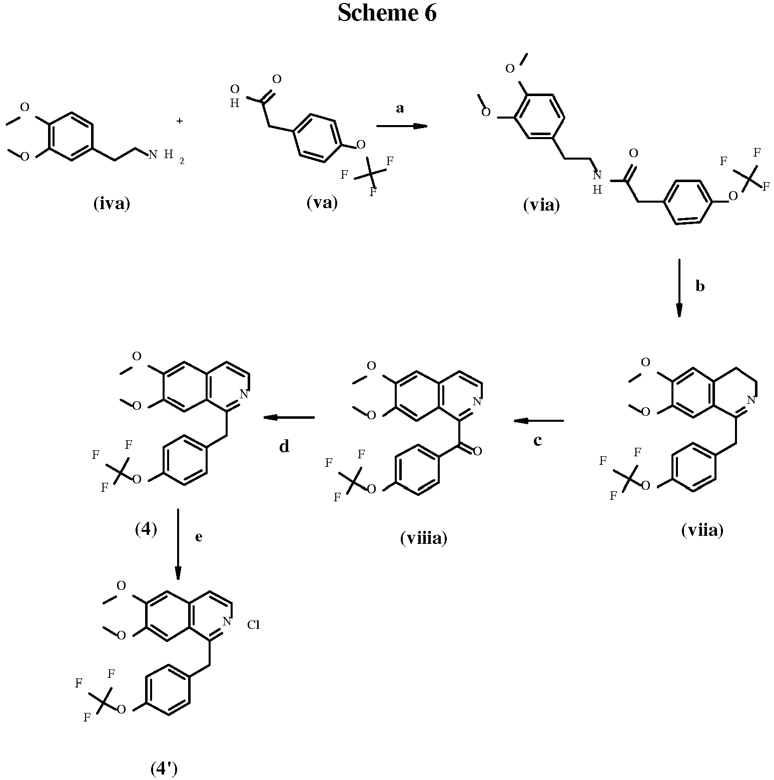

### c. Oxidation aromatization reaction

To the dihydroisoquinoline intermediate of Formula **(viia)** (m=750 mg, 2.0 mmol) in toluene (18 ml) were added MnO₂ (3.7 g, 42.6 mmol) and Na₂SO₄ (4.5 g, 31.7 mmol). The whole is heated 2h at reflux. After cooling and filtration, the crude is separated on silica gel (Eluent CH₂Cl₂/MeOH - 99/1) to lead to a carbonyl intermediate of Formula **(viiia)** (m=270 mg, 35%) characterized as follows: ¹H NMR (400 MHz, CDCl₃) δ(ppm) 8.47 (d, *J* = 5.5 Hz, 3H), 8.05 (d, *J* = 8.9 Hz, 6H), 7.73 (s, 2H), 7.69 (s, 2H), 7.32 (dd, *J* = 8.9, 0.9 Hz, 6H), 7.27 (s, 2H), 7.16 (s, 3H), 4.07 (s, 9H), 4.00 (s, 9H).

### d. Carbonyl reduction

To carbonyl intermediate of Formula **(vii)** (m=270 mg, 0.71 mmol) in glycol ethylene (10 ml) was added hydrazine monohydrate (0.15 ml, 3.1 mmol). The whole was heated 30 min at 120°C. After KOH (320mg - 5.7 mmol) in glycol ethylene (2.5 ml) was added. The whole was heated 3h at 190°C. After cooling and extraction with dichloromethane (3x3 5 ml), the organic layer is washed successively by water and bride. After drying and evaporation under reduce pressure, the crude is separated on silica gel under nitrogen (Eluant CH₂Cl₂/MeOH: 99/1) to lead to compound of the invention **(4)** (m=136 mg, 52.8%).

### d. Hydrochloride formation

β-carboline compound **(4)** (m=130 mg, 0.36 mmol) is solubilized at 0°C in an AcOEt (3 ml) and HCl (2M, in ethyl acetate, 1 ml) added. After 15 minutes agitation, solvent is removed under reduced pressure to lead to compound of the invention **(4')** (110 mg - 76.4%) characterized as follows: ¹H NMR (400 MHz, DMSO) δ(ppm) 8.43 (d, *J* = 6.5 Hz, 1H), 8.19 (d, *J* = 6.5 Hz, 1H), 7.84 (s, 1H), 7.78 (s, 1H), 7.68 (d, *J* = 8.7 Hz, 2H), 7.34 (d, *J* = 8.0 Hz, 2H), 5.10 (s, 2H), 4.04 (s, 3H), 4.00 (s, 3H). ¹³C NMR (101 MHz, DMSO) δ(ppm) 157.00, 153.80, 152.65, 147.82, 137.16, 136.39, 131.24, 130.30, 122.46, 122.32, 121.89, 107.20, 105.75, 57.15, 56.99, 35.10. DEPT 135 NMR (101 MHz, DMSO) δ(ppm) 131.24, 130.30, 122.46, 121.89, 107.20, 105.75, 57.16, 56.99, 35.10.

### Example 2: In vitro effects of compounds of the invention in enhancing lactate levels

To assess for the effect of the compounds of the invention on secretion of lactate, they have been tested in the following assays.

### Cell cultures

Primary cultures of cerebrocortical astrocytes were obtained from 1 to 2-day-old OF1 mouse pups (Charles River). Briefly, cortices were isolated and minced in small pieces under a dissecting microscope. The cells were incubated for 30 min at 37°C in a solution containing 20 U/ml papain, 1 mM L-cysteine and 10kU/ml DNase I. After dissociation, papain activity was stopped by the addition of fetal calf serum (FCS). Single-cell suspension was then obtained by mechanical dissociation, which consisted in cells trituration in a DMEM D7777 medium supplemented with 44 mm NaHCO₃, 10 ml/L antibiotic/antimycotic solution and 10% FCS. The cells were seeded at an average density of 6 × 10⁴ cells/cm² on poly-D-lysine-coated 96- or 12-well culture plates, depending on their use, and grown in DMEM D7777 medium supplemented with 44 mm NaHCO₃, 10 ml/L antibiotic/antimycotic solution and 10% FCS at 37°C in a humidified atmosphere containing 5% CO₂ / 95% air. Culture medium was renewed twice a week. Cells were stimulated and harvested between DIV14 and DIV17, when confluence and cell growth were optimal.

### Extracellular lactate quantification

Secretion of L-lactate was measured in the extracellular medium of 96-well plated astrocytes after 90 min stimulation (at 37°C, in 5% CO₂ / 95% air conditions) with Vehicle (DMSO), the compounds of the invention (100 nM to 100 µM) or positive control. The positive control consisted in Carbonyl cyanide m-chlorophenyl hydrazine (CCCP, 2 µM), an inhibitor of mitochondrial oxidative phosphorylation that hence lead to enhanced glycolysis and secretion of lactate. Stimulation medium was composed of D5030 medium complemented with 5 mM D-glucose and 44 mM sodium bicarbonate, pH 7.2. To quantify lactate concentrations in the extracellular medium, 200 µl of a 0.2M Glycine-semicarbazide buffer (pH 10) containing 3mM NAD and 14 U/ml LDH was added to each well of a 96-well plate containing 30µl aliquots of extracellular medium. Samples were incubated at 37°C for 1h. Fluorescence intensity (340 nm excitation/450 nm emission), which represents the amount of NADH produced, was measured, and lactate concentration values were determined relative to a standard curve of L-lactate concentrations.

The release of lactate from primary mouse astrocytes treated with compound **(1)** at 10 µM was quantified by (Fig. 1). Accumulation of lactate in the extracellular medium was measured over a period of 1.5h. Compounds **(1)** to **(4)** were tested at different concentrations ranging from 1 nM to 100 µM to determine their EC₅₀ and minimal effective concentrations and compound **(1)** has an EC₅₀ of 7.023 µM, while an effect already significant at concentrations below 1 µM could be monitored. Compounds **(2)** to **(4)** had effects on astrocyte-mediated lactate secretion of similar ranges, with EC₅₀ of 10.1 µM **(2**), 10.5 µM **(4)** and 10.9 µM **(3)***.* Maximal effects of the compounds were 38.6% **(1)**, 38% **(2)**, 31% **(4)** and 76% **(3)** when compared to the effect of the positive control (100%) for compounds **(1)** to **(4)**, respectively.

### Intracellular glycogen quantification

Astrocytes grown on 12-well plates were used for intracellular glycogen quantifications. Cells were stimulated with Vehicle (DMSO), with compounds of the invention (10 µM), or with a positive control for 180 min, at 37°C 5% CO₂/95% air in D5030 medium complemented with 5 mM D-glucose and 44 mM sodium bicarbonate (pH 7.2). Positive control consisted in an activator of glycogen phosphorylase, which hence triggers glycogen degradation in astrocytes (10 µM). At the end of the stimulation, medium was removed and replaced with 600 µl of 30 mM Tris HCl, and stored at -20°C.

First, the amount of proteins in each sample was quantified to assess whether harvested astrocytes from primary cell cultures yielded enough and equivalent amounts of proteins on each replicates. Proteins were quantified using the micro BCA Protein Assay kit (Thermo Scientific), according to manufacturer's instructions. Next, intracellular glycogen concentrations were quantified using a 250 µl-aliquot of the same stimulated, thawed, and sonicated cell lysate. After an incubation period of 30 min at 90°C and 400 rpm, 28 µl of a 0.1M acetic acid/sodium acetate buffer (pH 4.6) was added to each lysate aliquots, which were then separated in two. Each split aliquots received either 5 µl of amyloglucosidase or H₂O, and incubated for 120 min at 37°C. After centrifugation at 16'000G for 5min, 20 µl of supernatant were placed in a 96-well plate, to which 150 µl of a mix containing 0.67 mM ATP, 0.67 mM NADP, 1.8% hexokinase/glucose-6-phosphate dehydrogenase in a 0.1M Tris Buffer-HCl/3.3mM magnesium (pH 8.1) buffer was added. Fluorescence intensity (340 nm excitation/440 nm emission) was measured using a Safire 2 spectrophotometer. Glucose concentrations were assessed relative to a glucose standard curve, and glycogen concentrations were calculated by subtracting glucose values of samples that had received amyloglucosidase (i.e. that had degraded their glycogen stores) to samples that had not. Intracellular levels of glycogen, which is the main source of glucose storage in the brain, were analyzed in primary astrocytes after treatment with compound **(1)** (10 µM, 3h) (Fig. 2) and it was observed that compound **(1)** significantly enhances the degradation of intracellular glycogen, which may act, at least in part, as the energy fuel necessary to produce lactate by astrocytes during the process of aerobic glycolysis.

### MTT Mitochondrial activity assay

To monitor mitochondrial activity in astrocytes, which is linked to the metabolic process of glycolysis and production of lactate, astrocytes in 96-well plates were stimulated for 24h (37°C 5% CO₂/95% air) with the compounds of the invention ranging from 0.2 to 200 µM. After stimulation, 5 mg/ml thiazol blue tetrazolium bromide (MTT) in D5030 medium complemented with 5 mM D-glucose and 44 mM sodium bicarbonate (pH 7.2) was added to each well, and cells were incubated for 4h at 37°C (5% CO₂). The medium was then removed and the amount of reduced MTT, i.e formazan, solubilized in DMSO (50 µl/well) was determined using a spectrophotometer (absorbance of 570 nm).

The mitochondrial activity in primary astrocytes treated with Compounds **(2)** to **(4)** at concentrations ranging from 1 nM to 200 µM. After 24h, mitochondrial activity was monitored in MTT colorimetric assay as described above (Fig. 3). IC₅₀ were 34.4 µM (1), 42.9 µM **(2)**, 5.5 µM **(4)** and 26.6 µM **(3)**. Remaining mitochondrial activity at maximum concentrations of the compounds were 8.05% **(1)**, 7.86% **(2)**, 77.3% **(4)** and 35.9% **(3)** compared to vehicle respectively.

Altogether, those data support that compounds of the invention stimulate lactate secretion and glycogenolysis by astrocytes *in vitro.*

### Example 3: In vivo effects of compounds of the invention

To assess for the effect of the compounds of the invention on brain extracellular levels of lactate, they have been tested though the *in vivo* monitoring of lactate levels after treatment with the compounds of the invention as follows. Further, to assess for the effect of the compounds of the invention may have an effect in neurodegenerative disorder, since production of lactate in the brain is considered as being a key element underlying neuroprotection in neurodegenerative disease, including ALS (*Lee et al., 2012, supra;* Finsterwald et al., 2015, Curr. Drug Targets, 21(25):3570-81) they have been tested though in a mouse model of ALS as follows. Finally, in order to document the role of compounds of the invention on long-term memory, knowing that the production of lactate is a key element underlying synaptic plasticity and memory consolidation (*Suzuki et al, 2011, supra; Yang et al., 2014, supra; Tadi et al., 2015, supra*), they have been tested in an inhibitory avoidance (IA) test as described below.

All experiments were carried out in strict accordance with the Swiss Federal Guidelines for Animal Experimentation and were approved by the Cantonal Veterinary Office for Animal Experimentation (Canton of Vaud or Canton of Geneva, Switzerland).

For pharmacodynamics (Fig. 4) and cognition (Fig. 6) experiments, adult male C57Bl/6J wild-type mice weighting 18-28g (8 weeks of age) were used (Charles River). For ALS mouse models (Fig. 5), G93A SOD1 transgenic male and female mice on B6.SJL1-Gur/J background were used (Jackson Laboratory).

Animals were housed in groups of 3-5 in polypropylene cages (30 X 40 X 15 cm) with wire mesh top in a temperature (22 ± 2 °C) and humidity (55 ± 15%) controlled environment on a 12 hour light cycle (07.00-19.00h lights on), except after surgeries when animal were housed individually. The samples (Vehicle or compounds of the invention) were administered *per os* (gavage) in a solution made of water supplemented with 0.4% hydroxypropyl methylcellulose (HPMC) Methocel 4KM (w/v) and 0.25% Tween-20 (v/v), as previously described (Thackaberry et al., 2010, Toxicol Sci., 117(2):485-92). Concentrations of the compounds tested ranged from 10 to 100 mg/kg.

### In vivo pharmacodynamics - Lactate biosensors

Cerebral extracellular levels of lactate were monitored *in vivo* using lactate biosensors (Pinnacle Technology), according to the manufacturer's instructions. Cannulae were surgically implanted in the cerebral motor cortex areas M1/M2 (coordinates: +1.94 mm (to bregma), lateral -1.4 mm (to mideline), ventral -1.0mm (to dura)) of isoflurane-anesthesized mice 5 to 7 days prior experiment. After surgery, mice were monitored closely and received analgesic treatment for at least 4 days. After mice had fully recovered from surgery, compounds of the invention of vehicle were administered *per os* as previously described and cerebral levels of extracellular lactate were dynamically recorded for 6 hours using lactate biosensors. Mice were administered vehicle alone first, followed 3 hours later by vehicle or Compound **(1)** (10 or 100 mg/kg). Concentrations of cerebral extracellular lactate were calculated from lactate probe electric signals using post-calibration values. Each signal of lactate fluctuation after compound (or vehicle) administration was expressed as a fold change relative to the lactate fluctuation following the first administration of vehicle alone, each animal hence being its own control. Area Under the Curve (AUC) of lactate concentration curves were calculated using Graphad Prism and the ratio of AUC after drug over Vehicle administration was calculated.

Extracellular concentrations of L-lactate were measured in real time in freely moving animals for 3 hours after administration of Vehicle or Compound **(1)** (Fig. 4). Results indicate that treatment with Compound **(1)** at 10 mg/kg and 100 mg/kg significantly increases extracellular lactate levels in the brain of treated mice, as compared to vehicle (Fig. 4 C-F).

### SOD1 G93A mouse ALS model

Transgenic mice on B6.SJL1-Gur/J background overexpressing the human mutated gene G93A SOD1 were used. Mating colonies were composed of wild-type female mice and SOD1 G93A male mice, both on B6.SJL1-Gur/J background (purchased from Jackson Laboratory). F1 pups were genotyped after ear punching at weaning, using quantitative PCR (qPCR), which allowed determining the number of SOD1 copies in each mouse.

To test for the potential therapeutic effect of the administered compound, SOD1 mice were given orally the compound of the invention (10 mg/kg) or vehicle every day from post-natal day 30 (weaning) throughout their entire life. 3 groups were compared: wild-type mice treated with Vehicle, G93A SOD1 mice treated with Vehicle and G93A SOD1 mice treated with the compound of the invention. Each mouse's weight was recorded every day throughout the entire treatment, while neuromuscular function was measured once a week. Evaluation of neuromuscular function consisted in testing muscle strength using the grip test described below.

### Grip test

The experiment was conducted in a room with a low light intensity (30 lux) to reduce stressful environment. Mice were individually placed upside-down on the centre of a 35cm-high 42x42cm grid, which was placed on a bubble pack-lined table, for a maximal period of 5min. Mice ability to grip the grid (time [s]) was measured in order to assess for their muscle strength and coordination. Each mouse was tested on 3 consecutive trials with at least 20 min intervals between trials, and maximal value among the 3 trials was used.

### Survival

Mice were sacrificed when they reached at least one of the predefined criteria: i) lost of ≥15% of their maximal weight, ii ≥ 20s to move back when placed on their back (maximum criteria on the paralysis evaluation scale). Kaplan-Meier survival curves were then compared using Graphpad prism v.6.

Mice overexpressing mutated SOD1 G93A were treated with Vehicle or compound **(1)** by oral administration every day from weaning (post-natal day 30) to final stage (complete paralysis of rear paws). Every week, muscle function was monitored using a grip test. Data indicate that treatment with compound **(1)** delayed the onset of the symptoms, which remained significantly improved as long as the muscle function was measured until 16 weeks of age (Fig. 5A). In addition, treatment with compound **(1)** significantly increased life span of mice compared to treatment with vehicle alone (Fig. 5B), thereby providing in vivo support for the neuroprotective effect of the compounds of the invention in a neurodegenerative mouse model.

### Long-term memory test-Inhibitory Avoidance (IA)

IA test is a well-established memory paradigm in rodents that measures contextual memory associated with a mild electrical footshock in a specific context (the dark compartment of the IA chamber). Groups of 8-week old C57Bl/6 wild-type female mice were tested. Each mouse was handled for 5 minutes per day for at least 4 consecutive days to reduce animal's stress due to experimenter's presence/manipulation during test days. Inhibitory avoidance was carried out in an IA chamber (MedAssociates) that consists in a rectangular Perspex box divided into a safe and a shock compartment separated by an automatically operated sliding door. The safe compartment is white and illuminated while the shock compartment is black and dark. Mice were trained for IA 20 min after oral administration of the drug or vehicle. During training, mice were placed into the safe compartment with their heads facing away from the door. After 10 seconds, the door separating the compartments was automatically opened, allowing the mouse to access the shock compartment (which it usually did within 20 sec). The door closed 1 second after the mouse entered the dark compartment, and a 2-second 0.6 mA intensity footshock was delivered to the grid floor of the shock chamber via a constant current scrambler circuit. After footshock delivery, mice stayed for 10 seconds in the dark compartment and were then returned to their home cages. Memory retention was measured at 24h or 3 weeks after training by placing the mouse back into the lit compartment and recording its latency (in seconds) to enter the dark compartment. No footshock was administered during retention tests. The test was terminated once the mouse entered the dark compartment, or after a 900 seconds cutoff limit.

Statistical analyses were done using Graphpad prism v.6 using unpaired or paired 2-way Student's t-test for pairwise comparisons, or one-way ANOVA followed by Dunnett or Bonferroni post-hoc tests (Ludbrook, 1998, Clin Exp Pharmacol Physiol, 25(12):1032-7) when appropriate for multiple pair-wise comparisons.

Memory was measured after oral administration of vehicle or compound **(1)** (100 mg/kg) to young adult male mice orally, and 20 min later a training for IA (Fig. 6). 24 hours and 3 weeks after training, latencies of mice to enter back into the dark compartment of the IA chamber, the place where they received the shock, were measured. Results indicate that all mice had same latencies at training (no motor or cognitive differences), while their latencies were significantly higher when treated with compound **(1)** compared to vehicle at both 24h hours and 3 week-time points (Fig. 6C). These data indicate that treatment with a single dose of compound **(1)** (100 mg/kg) before training enhances long-term memory consolidation and/or expression.

## Claims

1. A compound of Formula (I), 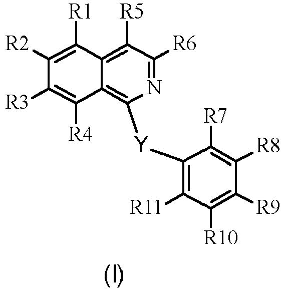 wherein R¹, R², R³, R⁴, R⁵ and R⁶ are independently selected from H, halogen, optionally substituted alkoxy, optionally substituted C₁-C₆ alkyl, optionally substituted amine, optionally substituted carboxylic acid or ester, nitro and nitrile; R⁷, R⁸, R⁹, R¹⁰ and R¹¹ independently selected from H, halogen, optionally substituted alkoxy, optionally substituted C₁-C₆ alkyl, optionally substituted amine, optionally substituted carboxylic acid or ester, nitro and nitrile and a group of Formula **(II):** -(X)ₘ-CR¹²R¹³R¹⁴ **(II)** wherein at least one group of R⁷, R⁸, R⁹, R¹⁰ and R¹¹ is a group of Formula **(II)**; X is selected from O, NR¹⁵ , S, CH₂ and hydrazine (-N-N-), m is an integer elected from 0 and 1 and R¹², R¹³ and R¹⁴ are independently selected from H, OH, optionally substituted alkoxy, optionally substituted C₁-C₆ alkyl and halogen, wherein at least one of R¹², R¹³ and R¹⁴ is F or Cl; Y is selected from -CR¹⁶R¹⁷- and -NR¹⁸-; R¹⁵ is selected from H, optionally substituted alkoxy and optionally substituted C₁-C₆ alkyl; R¹⁶ and R¹⁷ are independently selected from H, halogen, optionally substituted alkoxy, optionally substituted C₁-C₆ alkyl and optionally substituted aryl; R¹⁸ is independently selected from H or optionally substituted C₁-C₆ alkyl; any pharmaceutically acceptable salts, hydrates, solvates, or polymorphs, tautomers, geometrical isomers, optically active forms, enantiomeric mixtures thereof, and mixtures thereof for use in the prevention, the repression or treatment of a disease or disorder related to a deficiency in energy metabolism in the central nervous system and/or a neurological disorder, in particular amyotrophic lateral sclerosis (ALS), dementia - in particular Alzheimer's disease, frontotemporal dementia (FTD), dementia with Lewy bodies (LBD) -, Parkinson's disease, multiple sclerosis, stroke, traumatic brain injury or a psychotic disorder such as depression, schizophrenia, mild cognitive impairments and epilepsy and for enhancing cognitive and memory functions.

2. A compound for use according to claim 1 wherein X is selected from O, NR¹⁵, S and hydrazine (-N-N-).

3. A compound for use according to claim 1 or 2 wherein Y is -NR¹⁸.

4. A compound for use according to claim 1 or 2 wherein Y is -CR¹⁶R¹⁷.

5. A compound for use according to any one of claims 1 to 4 wherein R¹, R⁴, R⁵ and R⁶ are H.

6. A compound for use according to any one of claims 1 to 5 wherein R² and R³ are selected from H and optionally substituted alkoxy such as methoxy.

7. A compound for use according to any one of claims 1 to 6 wherein m is 1.

8. A compound for use according to any one of claims 1 to 7 wherein R⁹ is a group-(X)ₘ-CR¹²R¹³R¹⁴.

9. A compound for use according to any one of claims 1 to 7 wherein X is O.

10. A compound for use according to any one of claims 1 to 9 wherein R¹², R¹³ and R¹⁴ are F.

11. A compound for use according to any one of claims 1 to 10 wherein R⁷, R⁸, R¹⁰ and R¹¹ are H.

12. A compound for use according to any one of claims 1 to 11 wherein R⁹ is OCF₃ or CF₃.

13. A pharmaceutical composition comprising a compound of Formula (I) 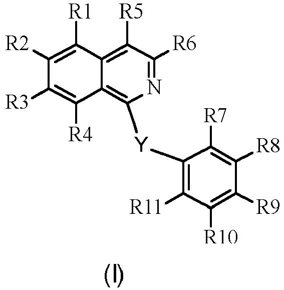 Wherein R¹, R², R³, R⁴, R⁵ and R⁶ are independently selected from H, halogen, C₁-C₆ alkyl, optionally substituted C₁-C₆ alkoxy, optionally substituted amine, nitro and nitrile, wherein C₁-C₆ alkoxy is optionally substituted by halogen; R⁷, R⁸, R⁹, R¹⁰ and R¹¹ independently selected from H, halogen, optionally substituted alkoxy, optionally substituted C₁-C₆ alkyl, optionally substituted amine, optionally substituted carboxylic acid or ester, nitro and nitrile and a group of Formula **(II):** -(X)ₘ-CR¹²R¹³R¹⁴ **(II)** wherein at least one group of R⁷, R⁸, R⁹, R¹⁰ and R¹¹ is a group of Formula **(II)**; X is selected from O, NR¹⁵, S, CH₂ and hydrazine (-N-N-), m is an integer elected from 0 and 1 and R¹², R¹³ and R¹⁴ are independently selected from H, OH, optionally substituted alkoxy, optionally substituted C₁-C₆ alkyl and halogen, wherein at least one of R¹², R¹³ and R¹⁴ is F or Cl; Y is selected from -CR¹⁶R¹⁷- and -NR¹⁸-; R¹⁵ is selected from H, optionally substituted alkoxy and optionally substituted C₁-C₆ alkyl; R¹⁶ and R¹⁷ are independently selected from H, halogen, optionally substituted alkoxy, optionally substituted C₁-C₆ alkyl and optionally substituted aryl; R¹⁸ is independently selected from H or optionally substituted C₁-C₆ alkyl; any pharmaceutically acceptable salts, hydrates, solvates, or polymorphs, tautomers, geometrical isomers, optically active forms, enantiomeric mixtures thereof, and mixtures thereof, and a pharmaceutically acceptable carrier, diluent or excipient thereof, with the proviso that it is not a compound selected from the following list:
N-[2-(trifluoromethyl)phenyl]isoquinolin-1-amine;
N-[3-(trifluoromethyl)phenyl]isoquinolin-1-amine; and
N-[4-(trifluoromethyl)phenyl]isoquinolin-1-amine.

14. A compound selected from the following group
6,7-dimethoxy-N-(4-(trifluoromethoxy)phenyl)isoquinolin-1-amine;
6,7-dimethoxy-N-(4-(trifluoromethyl)phenyl)isoquinolin-1-amine;
N-(4-(trifluoromethoxy)phenyl)isoquinolin-1-amine; and
6,7-dimethoxy-1-(4-(trifluoromethoxy)benzyl)isoquinoline; as well as tautomers, geometrical isomers, optically active forms, enantiomeric mixtures thereof, pharmaceutically acceptable salts and mixtures thereof.

15. A pharmaceutical composition according to claim 13, said composition comprising at least one compound according to claim 14.

16. A compound according to any one of claim 14 for use as a medicament.

17. A compound for use according to any one of claims 1 to 12, wherein said compound is according to claim 14.

## Patentansprüche

1. Verbindung der Formel (I), 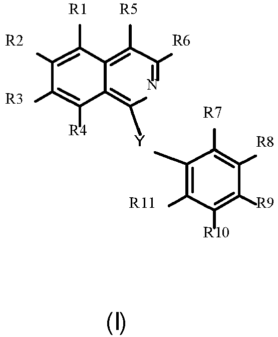 wobei R¹, R², R³, R⁴, R⁵ und R⁶ unabhängig aus H, Halogen, optional substituiertem Alkoxy, optional substituiertem C₁-C₆-Alkyl, optional substituiertem Amin, optional substituierter Carbonsäure oder optional substituiertem Carbonsäureester, Nitro und Nitril ausgewählt sind; R⁷, R⁸, R⁹, R¹⁰ und R¹¹ unabhängig aus H, Halogen, optional substituiertem Alkoxy, optional substituiertem C₁-C₆-Alkyl, optional substituiertem Amin, optional substituierter Carbonsäure oder optional substituiertem Carbonsäureester, Nitro und Nitril und einer Gruppe der Formel (**II**) ausgewählt sind: - (X)ₘ-CR¹²R¹³R1⁴ (**II**), wobei mindestens eine Gruppe von R⁷, R⁸, R⁹, R¹⁰ und R¹¹ eine Gruppe der Formel (**II**) ist; X aus 0, NR¹⁵, S, CH₂ und Hydrazin (-N-N-) ausgewählt ist, m eine ganze Zahl ist, die aus 0 und 1 ausgewählt ist, und R¹², R¹³ und R¹⁴ unabhängig aus H, OH, optional substituiertem Alkoxy, optional substituiertem C₁-C₆-Alkyl und Halogen ausgewählt sind, wobei mindestens eines von R¹², R¹³ und R¹⁴ F oder Cl ist; Y aus -CR¹⁶R¹⁷- und -NR¹⁸- ausgewählt ist; R¹⁵ aus H, optional substituiertem Alkoxy und optional substituiertem C₁-C₆-Alkyl ausgewählt ist; R¹⁶ und R¹⁷ unabhängig aus H, Halogen, optional substituiertem Alkoxy, optional substituiertem C₁-C₆-Alkyl und optional substituiertem Aryl ausgewählt sind; R¹⁸ unabhängig aus H oder optional substituiertem C₁-C₆-Alkyl ausgewählt ist; beliebige pharmazeutisch annehmbare Salze, Hydrate, Solvate oder Polymorphe, Tautomere, geometrische Isomere, optisch aktive Formen, Enantiomerenmischungen davon und Mischungen davon zur Verwendung bei der Prävention, Hemmung oder Behandlung einer Krankheit oder Erkrankung, die mit einem Defizit des Energiestoffwechsels im Zentralnervensystem zusammenhängt, und/oder einer neurologischen Erkrankung, insbesondere amyotrophe Lateralsklerose (ALS), Demenz - insbesondere Alzheimer-Krankheit, frontotemporale Demenz (FTD), Lewy-Body-Demenz (LBD) -, Parkinson-Krankheit, multiple Sklerose, Schlaganfall, Schädel-Hirn-Trauma, oder einer psychotischen Störung, wie Depression, Schizophrenie, leichte kognitive Beeinträchtigungen und Epilepsie, und zur Verbesserung der kognitiven und Gedächtnisfunktionen.

2. Verbindung zur Verwendung nach Anspruch 1, wobei X aus 0, NR¹⁵, S und Hydrazin (-N-N-) ausgewählt ist.

3. Verbindung zur Verwendung nach Anspruch 1 oder 2, wobei Y -NR¹⁸ ist.

4. Verbindung zur Verwendung nach Anspruch 1 oder 2, wobei Y -CR¹⁶R¹⁷ ist.

5. Verbindung zur Verwendung nach einem der Ansprüche 1 bis 4, wobei R¹, R⁴, R⁵ und R⁶ H sind.

6. Verbindung zur Verwendung nach einem der Ansprüche 1 bis 5, wobei R² und R³ aus H und optional substituiertem Alkoxy, wie Methoxy, ausgewählt sind.

7. Verbindung zur Verwendung nach einem der Ansprüche 1 bis 6, wobei m 1 ist.

8. Verbindung zur Verwendung nach einem der Ansprüche 1 bis 7, wobei R⁹ eine Gruppe - (X)ₘ-CR¹²R¹³R¹⁴ ist.

9. Verbindung zur Verwendung nach einem der Ansprüche 1 bis 7, wobei X O ist.

10. Verbindung zur Verwendung nach einem der Ansprüche 1 bis 9, wobei R¹², R¹³ und R¹⁴ F sind.

11. Verbindung zur Verwendung nach einem der Ansprüche 1 bis 10, wobei R⁷, R⁸, R¹⁰ und R¹¹ H sind.

12. Verbindung zur Verwendung nach einem der Ansprüche 1 bis 11, wobei R⁹ OCF₃ oder CF₃ ist.

13. Pharmazeutische Zusammensetzung, umfassend eine Verbindung der Formel (I): 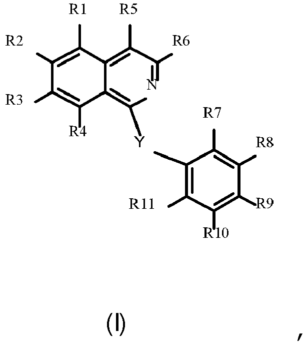 wobei R¹, R², R³, R⁴, R⁵ und R⁶ unabhängig aus H, Halogen, C₁-C₆-Alkyl, optional substituiertem C₁-C₆-Alkoxy, optional substituiertem Amin, Nitro und Nitril ausgewählt sind, wobei C₁-C₆-Alkoxy optional durch Halogen substituiert ist; R⁷, R⁸, R⁹, R¹⁰ und R¹¹ unabhängig aus H, Halogen, optional substituiertem Alkoxy, optional substituiertem C₁-C₆-Alkyl, optional substituiertem Amin, optional substituierter Carbonsäure oder optional substituiertem Carbonsäureester, Nitro und Nitril und einer Gruppe der Formel (**II**) ausgewählt sind: -(X)ₘ-CR¹²R¹³R1⁴ (**II**), wobei mindestens eine Gruppe von R⁷, R⁸, R⁹, R¹⁰ und R¹¹ eine Gruppe der Formel (**II**) ist; X aus 0, NR¹⁵, S, CH₂ und Hydrazin (-N-N-) ausgewählt ist, m eine ganze Zahl ist, die aus 0 und 1 ausgewählt ist, und R¹², R¹³ und R¹⁴ unabhängig aus H, OH, optional substituiertem Alkoxy, optional substituiertem C₁-C₆-Alkyl und Halogen ausgewählt sind, wobei mindestens eines von R¹², R¹³ und R¹⁴ F oder Cl ist; Y aus -CR¹⁶R¹⁷- und -NR¹⁸- ausgewählt ist; R¹⁵ aus H, optional substituiertem Alkoxy und optional substituiertem C₁-C₆-Alkyl ausgewählt ist; R¹⁶ und R¹⁷ unabhängig aus H, Halogen, optional substituiertem Alkoxy, optional substituiertem C₁-C₆-Alkyl und optional substituiertem Aryl ausgewählt sind; R¹⁸ unabhängig aus H oder optional substituiertem C₁-C₆-Alkyl ausgewählt ist; beliebige pharmazeutisch annehmbare Salze, Hydrate, Solvate oder Polymorphe, Tautomere, geometrische Isomere, optisch aktive Formen, Enantiomerenmischungen davon und Mischungen davon und einen pharmazeutisch unbedenklichen Trägerstoff, ein pharmazeutisch unbedenkliches Verdünnungsmittel oder einen pharmazeutisch unbedenklichen Hilfsstoff davon, mit der Maßgabe, dass sie keine Verbindung ist, die aus der folgenden Liste ausgewählt ist:
N-[2-(Trifluormethyl)phenyl] isochinolin-1-amin;
N-[3-(Trifluormethyl)phenyl] isochinolin-1-amin und
N-[4-(Trifluormethyl)phenyl] isochinolin-1-amin.

14. Verbindung, die aus der folgenden Gruppe ausgewählt ist:
6,7-Dimethoxy-N-(4-(trifluormethoxy)phenyl)isochinolin-1-amin;
6,7-Dimethoxy-N-(4-(trifluormethyl)phenyl)isochinolin-1-amin;
N-[4-(Trifluormethoxy)phenyl]isochinolin-1-amin; und
6,7-Dimethoxy-1-(4-(trifluormethoxy)benzyl)isochinolin;
sowie Tautomere, geometrische Isomere, optisch aktive Formen, Enantiomerenmischungen davon, pharmazeutisch unbedenkliche Salze und Mischungen davon.

15. Pharmazeutische Zusammensetzung nach Anspruch 13, wobei die Zusammensetzung mindestens eine Verbindung nach Anspruch 14 umfasst.

16. Verbindung nach Anspruch 14 zur Verwendung als ein Arzneimittel.

17. Verbindung zur Verwendung nach einem der Ansprüche 1 bis 12, wobei die Verbindung gemäß Anspruch 14 ist.

## Revendications

1. Composé de formule (I) 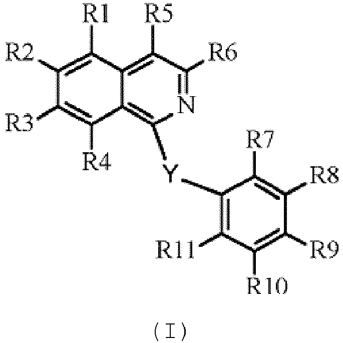 dans lequel R¹, R², R³, R⁴, R⁵ et R⁶ sont indépendamment choisis parmi H, un halogène, un alcoxy éventuellement substitué, un alkyle en C₁ à C₆ éventuellement substitué, une amine éventuellement substituée, un acide carboxylique ou un ester de celui-ci éventuellement substitué, nitro, et un nitrile ; R⁷, R⁸, R⁹, R¹⁰ et R¹¹ sont indépendamment choisis parmi H, un halogène, un alcoxy éventuellement substitué, un alkyle en C₁ à C₆ éventuellement substitué, une amine éventuellement substituée, un acide carboxylique ou un ester de celui-ci éventuellement substitué, nitro, un nitrile, et un groupe de formule (II) : -(X)ₘ-CR¹²R¹³R¹⁴ (II) pour lesquels au moins un groupe parmi R⁷, R⁸, R⁹, R¹⁰ et R¹¹ est un groupe de formule (II) ; X est choisi parmi 0, NR¹⁵, S, CH₂ et une hydrazine (-N-N-), m est un entier choisi parmi 0 et 1 et R¹², R¹³ et R¹⁴ sont indépendamment choisis parmi H, OH, un alcoxy éventuellement substitué, un alkyle en C₁ à C₆ éventuellement substitué et un halogène, pour lesquels au moins l'un parmi R¹², R¹³ et R¹⁴ est F ou Cl ; Y est choisi parmi -CR¹⁶R¹⁷- et -NR¹⁸- ; R¹⁵ est choisi parmi H, un alcoxy éventuellement substitué et un alkyle en C₁ à C₆ éventuellement substitué ; R¹⁶ et R¹⁷ sont indépendamment choisis parmi H, un halogène, un alcoxy éventuellement substitué, un alkyle en C₁ à C₆ éventuellement substitué et un aryle éventuellement substitué ; R¹⁸ est indépendamment choisi parmi H et un alkyle en C₁ à C₆ éventuellement substitué ; et n'importe quels sels, hydrates, solvates, ou polymorphes, tautomères, isomères géométriques, formes optiquement actives, mélanges d'énantiomères de ceux-ci, et mélanges de ceux-ci, pharmaceutiquement acceptables, pour une utilisation dans la prévention, la répression ou le traitement d'une maladie ou d'un trouble lié à une insuffisance du métabolisme énergétique dans le système nerveux central et/ou d'un trouble neurologique, en particulier une sclérose latérale amyotrophique(SLA), une démence - en particulier une démence d'Alzheimer, une démence frontotemporale (DFT), une démence à corps de Lewy (DCL) -, une maladie de Parkinson, une sclérose en plaques, un accident vasculaire cérébral, un traumatisme crânien ou un trouble psychotique tel qu'une dépression, une schizophrénie, des troubles cognitifs légers et une épilepsie, et pour amplifier les fonctions cognitives et mémorielles.

2. Composé pour une utilisation selon la revendication 1, dans lequel X est choisi parmi 0, NR¹⁵, S, et une hydrazine (-N-N-).

3. Composé pour une utilisation selon la revendication 1 ou 2, dans lequel Y est -NR¹⁸.

4. Composé pour une utilisation selon la revendication 1 ou 2, dans lequel Y est -CR¹⁶R¹⁷.

5. Composé pour une utilisation selon l'une quelconque des revendications 1 à 4, dans lequel R¹, R⁴, R⁵ et R⁶ sont H.

6. Composé pour une utilisation selon l'une quelconque des revendications 1 à 5, dans lequel R² et R³ sont choisis parmi H et un alcoxy éventuellement substitué tel que méthoxy.

7. Composé pour une utilisation selon l'une quelconque des revendications 1 à 6, dans lequel m vaut 1.

8. Composé pour une utilisation selon l'une quelconque des revendications 1 à 7, dans lequel R⁹ est un groupe -(X)ₘ-CR¹²R¹³R¹⁴.

9. Composé pour une utilisation selon l'une quelconque des revendications 1 à 7, dans lequel X est 0.

10. Composé pour une utilisation selon l'une quelconque des revendications 1 à 9, dans lequel R¹², R¹³ et R¹⁴ sont F.

11. Composé pour une utilisation selon l'une quelconque des revendications 1 à 10, dans lequel R⁷, R⁸, R¹⁰ et R¹¹ sont H.

12. Composé pour une utilisation selon l'une quelconque des revendications 1 à 11, dans lequel R⁹ est OCF₃ ou CF₃.

13. Composition pharmaceutique comprenant un composé de formule (I) 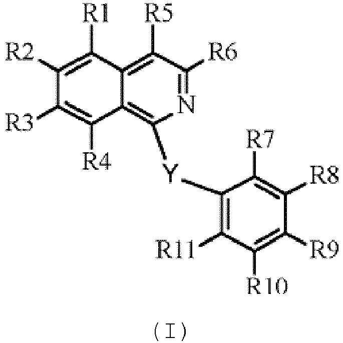 dans laquelle R¹, R², R³, R⁴, R⁵ et R⁶ sont indépendamment choisis parmi H, un halogène, un alkyle en C₁ à C₆ éventuellement substitué, un alcoxy en C₁ à C₆ éventuellement substitué, une amine éventuellement substituée, nitro, et un nitrile, parmi lesquels l'alcoxy en C₁ à C₆ est éventuellement substitué par un halogène ; R⁷, R⁸, R⁹, R¹⁰ et R¹¹ sont indépendamment choisis parmi H, un halogène, un alcoxy éventuellement substitué, un alkyle en C₁ à C₆ éventuellement substitué, une amine éventuellement substituée, un acide carboxylique ou un ester de celui-ci éventuellement substitué, nitro, un nitrile, et un groupe de formule (II) : -(X)ₘ-CR¹²R¹³R¹⁴ (II) pour lesquels au moins un groupe parmi R⁷, R⁸, R⁹, R¹⁰ et R¹¹ est un groupe de formule (II) ; X est choisi parmi 0, NR¹⁵, S, CH₂ et une hydrazine (-N-N-), m est un entier choisi parmi 0 et 1 et R¹², R¹³ et R¹⁴ sont indépendamment choisis parmi H, OH, un alcoxy éventuellement substitué, un alkyle en C₁ à C₆ éventuellement substitué et un halogène, pour lesquels au moins l'un parmi R¹², R¹³ et R¹⁴ est F ou Cl ; Y est choisi parmi -CR¹⁶R¹⁷- et -NR¹⁸- ; R¹⁵ est choisi parmi H, un alcoxy éventuellement substitué et un alkyle en C₁ à C₆ éventuellement substitué ; R¹⁶ et R¹⁷ sont indépendamment choisis parmi H, un halogène, un alcoxy éventuellement substitué, un alkyle en C₁ à C₆ éventuellement substitué et un aryle éventuellement substitué ; R¹⁸ est indépendamment choisi parmi H et un alkyle en C₁ à C₆ éventuellement substitué ; et n'importe quels sels, hydrates, solvates, ou polymorphes, tautomères, isomères géométriques, formes optiquement actives, mélanges d'énantiomères de ceux-ci, et mélanges de ceux-ci, pharmaceutiquement acceptables, et un véhicule diluant ou excipient pharmaceutiquement acceptable d'un tel composé, sous réserve qu'il ne s'agisse pas d'un composé choisi dans la liste suivante :
N-[2-(trifluorométhyl)phényl]isoquinolin-1-amine ;
N-[3-(trifluorométhyl)phényl]isoquinolin-1-amine ; et
N-[4-(trifluorométhyl)phényl]isoquinolin-1-amine.

14. Composé choisi dans le groupe suivant :
6,7-diméthoxy-N-(4-(trifluorométhoxy)phényl)isoquinolin-1-amine ;
6,7-diméthoxy-N-(4-(trifluorométhyl)phényl)isoquinolin-1-amine ;
N-(4-(trifluorométhoxy)phényl)isoquinolin-1-amine ; et
6,7-diméthoxy-1-(4-(trifluorométhoxy)benzyl)isoquinoline ;
ainsi que les tautomères, isomères géométriques, formes optiquement actives, mélanges d'énantiomères de ceux-ci, sels pharmaceutiquement acceptables et mélanges de ceux-ci.

15. Composition pharmaceutique selon la revendication 13, ladite composition comprenant au moins un composé selon la revendication 14.

16. Composé selon la revendication 14, pour une utilisation en tant que médicament.

17. Composé selon l'une quelconque des revendications 1 à 12, lequel composé est conforme à la revendication 14.
